# EUROPEAN PATENT SPECIFICATION

(11) **EP 2 201 116 B1**
(45) Date of publication and mention of the grant of the patent: **08.08.2012**
(21) Application number: 08802303.1
(22) Date of filing: 17.09.2008
(51) Int. Cl.: C12N 15/11, C12N 15/63, C12N 15/85, C12N 15/90, C12N 5/10, A01K 67/00, A61K 31/713

(54) **MEANS AND METHODS FOR SHRNA MEDIATED CONDITIONAL KNOCKDOWN OF GENES**
MITTEL UND VERFAHREN ZUM SHRNA-VERMITTELTEN KONDITIONALEN KNOCKDOWN VON GENEN
MOYENS ET PROCÉDÉS POUR UNE DÉSACTIVATION CONDITIONNELLE DE GÈNES À MÉDIATION PAR DE L'ARNSH

(30) Priority: 17.09.2007 US 973055 P
(43) Date of publication of application: 30.06.2010
(73) Proprietor: Helmholtz Zentrum München Deutsches Forschungszentrum für Gesundheit und Umwelt (GmbH), 85764 Neuherberg (DE)
(72) Inventor: KÜHN, Ralf, 85354 Freising (DE); WURST, Wolfgang, 80937 München (DE); STEUBER-BUCHBERGER, Patricia, 85298 Scheyern (DE)
(74) Representative: Vossius & Partner
(86) International application number: PCT/EP2008/007779
(87) International publication number: WO 2009/036971

(56) References cited:
- EP-A- 1 462 525
- LEE G ET AL: "Role of nucleotide sequences of loxP spacer region in Cre-mediated recombination" GENE, ELSEVIER, AMSTERDAM, NL, vol. 216, no. 1, 1 August 1998 (1998-08-01), pages 55-65, XP004149281 ISSN: 0378-1119
- LANGER STEPHEN J ET AL: "A genetic screen identifies novel non-compatible loxP sites" NUCLEIC ACIDS RESEARCH, vol. 30, no. 14, 15 July 2002 (2002-07-15), pages 3067-3077, XP002509092 ISSN: 0305-1048
- STEUBER-BUCHBERGER P ET AL: "Simultaneous cre-mediated conditional knockdown of two genes in mice" GENESIS 200803 US, vol. 46, no. 3, March 2008 (2008-03), pages 144-151, XP002509085 ISSN: 1526-954X 1526-968X

## Description

The present invention relates to a combination of DNA segments comprising: (a) a first segment comprising in 5' to 3' or 3' to 5' order: (aa) a promoter; (ab) a first DNA sequence comprising: (i) a DNA sequence giving rise upon transcription to the sense strand of an shRNA molecule; (ii) a transcriptional stop element which is flanked by a first type of recombinase recognition sequences; and (iii) a DNA sequence giving rise upon transcription to the antisense strand of an shRNA molecule; (b) a second segment comprising in 5' to 3' or 3' to 5' order: (ba) a promoter; (bb) a second DNA sequence comprising: (i) a DNA sequence giving rise upon transcription to the sense strand of an shRNA molecule; (ii) a transcriptional stop element which is flanked by a second type of recombinase recognition sequences; and (iii) a DNA sequence giving rise upon transcription to the antisense strand of an shRNA molecule; wherein (i) said first type of recombinase recognition sequences are recognized and recombined by a recombinase but not recombined with said second type of recombinase recognition sequences; (ii) said second type of recombinase recognition sequences are recognized and recombined by the recombinase of (i) but not recombined with said first type of recombinase recognition sequences; and (iii) said DNA sequence of (ab) and (bb) is expressed under the control of said promoters of (aa) and (ba) upon removal of said transcriptional stop elements of (ab) and (bb) by the activity of a recombinase, resulting in transcription of said shRNA molecule in a cell. Further, the invention relates to a genetically engineered non-human animal and a method to produce said transgenic non-human animal. Also, the invention relates to a cell genetically engineered with the DNA molecule of the invention and a method of simultaneously knocking down two genes in a cell. Furthermore, envisaged is a method of identifying a combination of two target genes as a potential drug target and the use of the DNA molecule of the invention for the preparation of a composition for gene therapy.

Several documents are cited throughout the text of this specification. RNA interference (RNAi) is a mechanism for RNA-guided regulation of gene expression and is conserved in most eukaryotic organisms. The RNAi pathway is thought to have evolved as a form of innate immunity against viruses and also plays a major role in regulating development and genome maintenance. In research, RNAi has become an extremely useful genetic tool to study gene function in mammalian cells. The discovery that short interfering (si)RNAs avoid an interferon response and the global shutdown of translation has enabled the wide use of transient gene silencing in cultured cells and specific tissues of mice upon local administration (Lieberman et al., (2003), Trends Mol. Med., 9, 397-403). To elicit permanent gene silencing, short hairpin (sh)RNA expression vectors can be used. These vectors consist of an RNA polymerase III promoter producing short RNA fragments, which form hairpin structures. Subsequently, the shRNAs are processed by the RNAi machinery resulting in siRNAs which subsequently mediate sequence-specific gene silencing (Dykxhoorn et al., (2003), Nat. Rev. Mol. Cell Biol., 4, 457-467).

The shRNA vectors have also been successfully used to produce an all-over knockdown phenotype similar to conventional knockout mice by creating mice transgenic for an shRNA vector (Kunath et al., (2003), Nat. Biotechnol., 21, 559-561). This method has proved to be useful in solving the problem of embryonic lethality arising from inter alia targeting of genes which are somehow involved in the developmental phase of the embryo. Conditional vectors allow for regulated expression of shRNA molecules and thus expression can be turned on leading to knockdown of the target gene in a tissue-specific and/or time dependent manner. Regulation can be achieved by using an inducing compound such as, for example, doxycycline which acts on artificial regulatory sequences in the polymerase III promoter (Chen et al., (2003), Cancer Res., 63, 4801-4804) or by lifting a blockade of transcription. Transcriptional stop elements can block transcription and can be excised using the Cre/loxP approach to allow for time- and/or tissue-specific knock down of a gene. Various vector designs for Cre/loxP mediated RNAi have been described (Kasim et al., (2004), Nucleic Acids Res., 32, e66; Tiscornia et al., (2004), Proc. Natl Acad Sci. USA, 101, 7347-7351), nevertheless, there is a steady demand for improved gene targeting and knockdown methods for use in a variety of fields.

The technical problem underlying the present invention was to identify alternative and/or improved means and methods that allow for gene knockdown.

The solution to this technical problem is achieved by providing the embodiments characterized in the claims.

Accordingly, the present invention relates in a first embodiment to a combination of DNA segments comprising:
(a) a first segment comprising in 5' to 3' or 3' to 5' order:
   (aa) a promoter;
   (ab) a first DNA sequence comprising:
      (i) a DNA sequence giving rise upon transcription to the sense strand of an shRNA molecule;
      (ii) a transcriptional stop element which is flanked by a first type of recombinase recognition sequences; and
      (iii) a DNA sequence giving rise upon transcription to the antisense strand of an shRNA molecule;
(b) a second segment comprising in 5' to 3' or 3' to 5' order:
   (ba) a promoter I;
   (bb) a second DNA sequence comprising:
      (i) a DNA sequence giving rise upon transcription to the sense strand of an shRNA molecule;
      (ii) a transcriptional stop element which is flanked by a second type of recombinase recognition sequences; and
      (iii) a DNA sequence giving rise upon transcription to the antisense strand of an shRNA molecule;
   wherein
   (i) said first type of recombinase recognition sequences are recognized and recombined by a recombinase but not recombined with said second type of recombinase recognition sequences;
   (ii) said second type of recombinase recognition sequences are recognized and recombined by the recombinase of (i) but not recombined with said first type of recombinase recognition sequences; and
   (iii) said DNA sequence of (ab) and (bb) is expressed under the control of said promoters of (aa) and (ba) upon removal of said transcriptional stop elements of (ab) and (bb) by the activity of a recombinase, resulting in transcription of said shRNA molecule in a cell.

The term "promoter" relates to promoters which are functional in a cell and mediate the expression of the shRNA molecule within said cell. The structure and function of prokaryotic and eukaryotic promoters are well-known to the person skilled in the art and described, for example, in "Molecular Cell Biology", Lodish et al. (eds), W.H.Freeman&Co, New York. The promoters may be RNA polymerase II or III dependent and constitutively active or inducible, ubiquitous or gene-/tissue-specific. Further, the promoters may contain artificially introduced sequences to modify their regulatory capacity, such as, for example, enhancers, silencers, insulators, specific transcription factor binding sites or specific operator sequences like to, tet, Gal4, lac conferring inducibility to said promoter. Preferred cells are eukaryotic cells. Accordingly, preferred are promoters which are functional in eukaryotic cells.

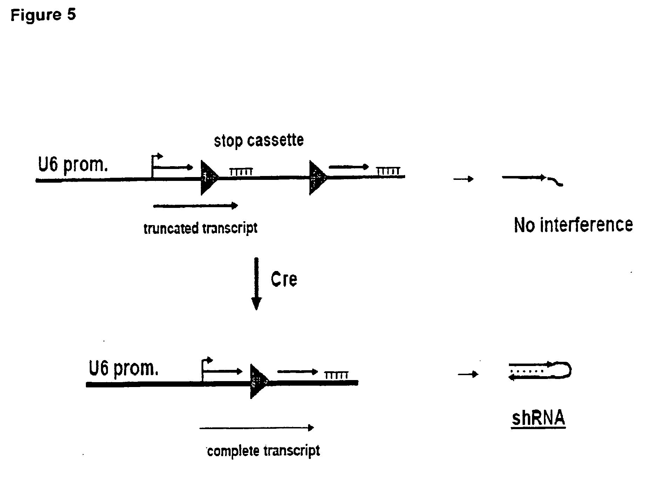

The terms "sense" and "antisense strand of an shRNA molecule" relates to the RNA strands which due to their complementary RNA sequence undergo basepairing and form the characteristic double-strand RNA segment of an shRNA molecule which is responsible for mediating RNA interference. The shRNA molecule generated in accordance with the invention is composed of a double stranded segment and further a single stranded segment commonly referred to as the "stem loop" or "hairpin" structure (cf. Figure 5). Said structure is the result of the basepairing of the complementary RNA sequences of the sense and antisense strand which flank a stretch of non-complementary nucleotides. The stem loop also comprises a lox sequence wherein said lox sequence is the result of the excision of the transcriptional stop element and the subsequent ligation of the DNA strand ends encoding the shRNA molecule.

The term "a transcriptional stop element" as used in accordance with the present invention relates to a transcriptional stop element which is located within the region of the DNA sequence that after excision constitutes the region of the shRNA molecule that does not contain base pairs complementary to each other thus not forming an RNA double strand, commonly referred to as the "loop". A transcriptional stop element may be any element which due to its nucleotide sequence leads to the arrest of transcription of functional, i.e. RNAi-mediating, shRNA molecules. Naturally occurring transcriptional stop elements are well-known in the art such as, for example, poly-adenylation signals in eukaryotes.

"Recombinase recognition sequences" as used in the present invention relate to sequences which are recognized by enzymes capable of mediating site-specific recombination. Recombination involves enzyme-mediated cleavage of a DNA double strand and subsequent ligation of the cleaved DNA double strand to either the same or another equally cleaved DNA strand. The recombinase recognition sequence has a sequence motif which is specifically recognized by a recombinase and further contains a sequence motif which matches exactly with the terminal end of a DNA molecule for subsequent ligation, wherein recognition and matching motif can be overlapping or be the same. Thus, recombination can be used to excise or introduce a DNA segment by cleavage and ligation of dsDNA. Preferred are recombination recognition sequences which are aligned such as to allow for excision of the transcriptional stop elements, i.e., for example, using loxP sequences as direct repeats instead of inverted repeats. The above applies mutatis mutandis to other embodiments herein.

The combination of DNA segments of the present invention relates to DNA segments which are suitable for efficient knockdown of target genes upon the excision of the transcriptional stop elements by the activity of a recombinase as described herein resulting in the transcription of shRNA molecules. Thus, said combination relates to DNA segments comprising DNA sequences which are accessible for the cellular transcriptional machinery leading to expression of shRNA molecules within a eukaryotic cell. The transcription process is preferably mediated by RNA polymerase III. The structure and function of short hairpin RNA molecules are well-known to the skilled person and methods for production and design are described, for example, in Paddison et al., (2002), Genes Dev., 16(8), 948-958; McIntyre et Fanning, (2006), BMC Biotechnol., 6:1.

Short hairpin RNA is capable of mediating gene knockdown just like any dsRNA in a process termed RNA interference (Fire et al., (1998), Nature, 391,806-811). Once an shRNA molecule is transcribed in the cell, it is cleaved by an RNase III-like enzyme (Dicer), into double stranded small interfering RNAs (siRNA) loosing its loop structure. In an ATP dependent step, the siRNAs become integrated into a multi-subunit protein complex, commonly known as the RNAi induced silencing complex (RISC), which guides the siRNAs to the target RNA sequence (Nykanen et al., (2001), Cell, 107, 309-321). At some point during the integration phase the siRNA duplex unwinds, and the antisense strand remains bound to RISC and directs degradation of the complementary mRNA sequence by a combination of endo- and exonucleases (Martinez et al., (2002), Cell, 110, 563-574).

Efficiency of the knockdown may be measured by methods well-known in the art, for example, by measuring the protein or mRNA levels of the target genes before and after knockdown or by reporter gene assays. Preferably, the efficiency of the knockdown of the target genes mediated by the DNA molecule of the present invention is similar or preferably equal for either of said genes and knocks down at least 10%, at least 20%, at least 30%, at least 40%, at least 50%, at least 60%, at least 70%, at least 80% and preferably at least 90% or 95% of the target gene product relative to a control. Most preferred a complete knockdown of the target genes is envisaged.

Although RNAi-mediated gene knockdown has been the focus of research in a variety of fields, the present investigators surprisingly show regulated, conditional and efficient expression of two different shRNA molecules transcribed from one DNA molecule leading to simultaneous knockdown of two genes. The novel technique allows for conditional expression of two or more shRNA molecules upon recombinase-mediated activation.

Exemplarily, the present inventors impaired expression of two different genes by coupling two short hairpin RNAs (shRNA), each specific for one gene, in a row in one expression vector. To achieve a conditional activation of the shRNAs they used the Cre/loxP site specific recombination system. The invention comprises additionally to the wild type loxP sites, a mutated lox site, called lox2272 (Araki et al., Nucleic Acids Res., 30 (19), e103 (2002)), which cannot recombine with the wild type loxP site. One of the used shRNAs is interrupted by a loxP flanked stop cassette and the second shRNA is interrupted by a lox2272 flanked stop cassette. Contact with Cre recombinase leads to recombination of the loxP sites in one shRNA and of the lox2272 in the other shRNA. In this recombination step the stop cassettes are cut out and RNAi is activated engaging the two targeted genes with the now active shRNAs. The activity pattern of Cre recombinase allows a control of the targeted tissues and cell types. By placing the conditional RNAi constructs into the defined genetic Rosa26 locus and by using recombinase mediated cassette exchange (RMCE) they were able to produce transgenic animals displaying a high rate of knockdown of the two targeted genes.

In conclusion, the present invention provides a technique for easy conditional silencing of two or multiple genes or gene families. This is important in a variety of fields, especially with regard to transgenic animals used as disease models or to generally assess gene function. Thus, an obvious application for the present invention is the production of shRNA vector mice using the large collection of mouse strains already carrying an expressible recombinase gene under the control of a tissue-specific promoter, for example, the Cre recombinase, which will allow for fast generation of a variety of different knockdown mouse strains as compared to the laborious, time-consuming method of generating traditional knockout mouse strains, which moreover only seldom are double knockouts. Furthermore, double knockouts cannot be produced within one step. Due to the compatibility of the combination of DNA segments with RMCE, i. e. recombination-mediated cassette exchange as described in detail further below herein, being a single-copy approach using defined and well-characterized genetic loci is available, which has the advantage of reliably generating double knockdown mice showing a well-definable, ubiquitous or tissue-specific knockdown phenotype as compared to knockout mice generated through homologous recombination.

In a preferred embodiment of the combination of DNA segments of the invention, the DNA segments are contained in the same DNA molecule.

In accordance with the invention said combination of DNA segments is preferably contained in the same DNA molecule, said segments being placed next to each other in the same or opposite direction. Said DNA molecule may contain further sequence elements flanking said combination of DNA segments of the invention. Such sequence elements can be, for example and without limitation, elements allowing for integration into another DNA molecule, such as, restriction sites or recombinase recognition sites.

In a preferred embodiment of the combination of DNA segments of the invention, said combination of DNA segments is part of a vector.

In accordance with the present invention, the DNA segments can be part of the same vector or can each be part of different vectors, wherein in the latter case both vectors have to be present at the same time to allow for simultaneous knockdown of two target genes. Preferably, each of the DNA segments is part of the same vector and arranged as described above when being contained in the same DNA molecule.

Preferably, the a vector is a plasmid, cosmid, virus, bacteriophage or another vector used, e.g., conventionally in genetic engineering.

The DNA segments may further be inserted into several commercially available vectors to be part thereof. Non-limiting examples include prokaryotic plasmid vectors, such as the pUC-series, pBluescript (Stratagene), the pET-series of expression vectors (Novagen) or pCRTOPO (Invitrogen) and vectors compatible with an expression in mammalian cells like pREP (Invitrogen), pcDNA3 (Invitrogen), pCEP4 (Invitrogen), pMC1neo (Stratagene), pXT1 (Stratagene), pSG5 (Stratagene), EBO-pSV2neo, pBPV-1, pdBPVMMTneo, pRSVgpt, pRSVneo, pSV2-dhfr, pIZD35, pLXIN, pSIR (Clontech), pIRES-EGFP (Clontech), pEAK-10 (Edge Biosystems) pTriEx-Hygro (Novagen), pbsU6 and pCINeo (Promega).

The DNA segments may also be inserted into vectors to be part thereof such that a translational fusion with another DNA molecule is generated. The other DNA molecule may encode another protein which may e.g. mediate the recombination event leading to the excision of the transcriptional stop elements.

For vector modification techniques, see Sambrook, Molecular Cloning, A Laboratory Manual, Cold Spring Harbor Laboratory (1989) N.Y. Generally, vectors can contain one or more origin of replication (ori) and inheritance systems for cloning or expression, one or more markers for selection in the host, e. g., antibiotic resistance, and one or more expression cassettes. Suitable origins of replication (ori) include, for example, the Col E1, the SV40 viral and the M 13 origins of replication.

The coding sequences inserted besides the DNA segments in the vector can e.g. be synthesized by standard methods, or isolated from natural sources. Ligation of the coding sequences to transcriptional regulatory elements and/or to other amino acid encoding sequences can be carried out using established methods. Transcriptional regulatory elements (parts of an expression cassette) ensuring expression eukaryotic cells are well known to those skilled in the art. These elements comprise regulatory sequences ensuring the initiation of the transcription (e. g., translation initiation codon, promoters, enhancers, and/or insulators), internal ribosomal entry sites (IRES) (Owens, Proc. Natl. Acad. Sci. USA 98 (2001), 1471-1476) and optionally poly-A signals ensuring termination of transcription and stabilization of the transcript. Additional regulatory elements may include transcriptional as well as translational enhancers, and/or naturally-associated or heterologous promoter regions. Preferably, said coding sequences are operatively linked to such expression control sequences allowing expression in prokaryotes or eukaryotic cells. The vector may further comprise nucleotide sequences encoding secretion signals as further regulatory elements. Such sequences are well known to the person skilled in the art. Furthermore, depending on the expression system used, leader sequences capable of directing the expressed polypeptide to a cellular compartment may be added to the coding sequence of the polynucleotide of the invention. Such leader sequences are well known in the art.

Possible examples for regulatory elements ensuring the initiation of transcription comprise the cytomegalovirus (CMV) promoter, SV40-promoter, RSV-promoter (Rous sarcome virus), the lacZ promoter, the gai10 promoter, human elongation factor 1α-promoter, CMV enhancer, CaM-kinase promoter, the Autographa californica multiple nuclear polyhedrosis virus (AcMNPV) polyhedral promoter, the SV40-enhancer or a U6 or H1 gene promoter. Examples for further regulatory elements of prokaryotes and eukaryotic cells comprise transcription termination signals, such as SV40-poly-A site or the tk-poly-A site or the SV40, lacZ and AcMNPV polyhedral polyadenylation signals, downstream of the polynucleotide.

Furthermore, it is preferred that the vector comprises a selectable marker. Examples of selectable markers include neomycin, ampicillin, and hygromycine, kanamycine resistance and the like. Specifically-designed vectors allow the shuttling of DNA between different hosts, such as bacteria-fungal cells or bacteria-animal cells (e. g. the Gateway® system available from Invitrogen).

An expression vector in accordance with this invention is capable of directing the replication, and the expression of the DNA segments of this invention encoding shRNA molecules. Suitable expression vectors which comprise the described regulatory elements are known in the art such as Okayama-Berg cDNA expression vector pcDV1 (Pharmacia), pRc/CMV, pcDNA1, pcDNA3 (In-Vitrogene), pSPORT1 (GIBCO BRL), or pGEMHE (Promega), or prokaryotic expression vectors, such as lambda gt11, pJOE, the pBBR1-MCS -series, pJB861, pBSMuL, pBC2, pUCPKS, pTACT1, pET vector (Novagen) or, preferably, expression vectors containing RNA Polymerase III driven promoters like pSUPER (Brummelkamp, et al., Science, 296, 550-553 (2002), pShag (Paddison, et al., Nat Methods, 1, 163-167 (2004) or the pBS-U6 vector (as used in the appended example).

The combination of DNA segments of the invention being part of the vectors as described herein above may be designed for direct introduction or for introduction via liposomes, phage vectors or viral vectors (e.g. adenoviral, retroviral) into the eukaryotic cell. Additionally, baculoviral systems or systems based on Vaccinia Virus or Semliki Forest Virus can be used as eukaryotic expression system for the combination of the DNA segments of the invention.

A typical mammalian expression vector may - besides the combination of DNA segments of the invention - further contain a promoter element, which mediates the initiation of transcription of mRNA, a protein coding sequence, and signals required for the termination of transcription and polyadenylation of the transcript. Moreover, elements such as origin of replication, drug resistance gene, regulators (as part of an inducible promoter) may also be included. Additional elements might include enhancers, Kozak sequences and intervening sequences flanked by donor and acceptor sites for RNA splicing. Highly efficient transcription can be achieved with the early and late promoters from SV40, the long terminal repeats (LTRs) from retroviruses, e.g., RSV, HTLVI, HIVI, and the early promoter of the cytomegalovirus (CMV). However, cellular elements can also be used (e.g., the human actin promoter). Suitable expression vectors for use in practicing the present invention include, for example, vectors such as pSVL and pMSG (Pharmacia, Uppsala, Sweden), pRSVcat (ATCC 37152), pSV2dhfr (ATCC 37146) and pBC12MI (ATCC 67109), IpSUPER (Brummelkamp, et al., Science, 296, 550-553 (2002)), pShag

(Paddison, et al., Nat Methods, 1, 163-167 (2004)) or the pBS-U6 vector (as used in the appended example; SEQ ID NO: 8). Mammalian host cells that could be used include, human Hela, 293, H9 and Jurkat cells, mouse NIH3T3 and C127 cells, Cos 1, Cos 7 and CV1, quail QC1-3 cells, mouse L cells and Chinese hamster ovary (CHO) cells. Alternatively, the shRNA molecules can be expressed in stable cell lines that contain the combination of DNA segments integrated into a chromosome. The co-transfection with a selectable marker such as dhfr, gpt, neomycin, hygromycin allows the identification and isolation of the transfected cells. The transfected nucleic acid can also be amplified to express large amounts of the encoded shRNAs. The DHFR (dihydrofolate reductase) marker is useful to develop cell lines that carry several hundred or even several thousand copies of the vectors carrying the combination of the DNA segments of the invention. Another useful selection marker is the enzyme glutamine synthase (GS) (Murphy et al.1991, Biochem J. 227:277-279; Bebbington et al. 1992, Bio/Technology 10:169-175). Using these markers, the mammalian cells are grown in selective medium and the cells with the highest resistance are selected. As indicated above, the expression vectors will preferably include at least one selectable marker. Such markers include dihydrofolate reductase, G418 or neomycin resistance for eukaryotic cell culture and tetracycline, kanamycin or ampicillin resistance genes for culturing in *E*. *coli* and other bacteria. Representative examples of appropriate hosts include, but are not limited to, bacterial cells, such as *E*. *coli,* Streptomyces and *Salmonella typhimurium* cells; fungal cells, such as yeast cells; insect cells such as Drosophila S2 and Spodoptera Sf9 cells; animal cells such as CHO, COS, 293 and Bowes melanoma cells; and plant cells. Appropriate culture mediums and conditions for the above-described host cells are known in the art.

In another preferred embodiment of the combination of DNA segments of the invention, the recombinase recognition sequences are lox sequences encompassing a wild type loxP sequence and a mutant loxP sequence.

The term "lox sequences" as used in accordance with the present invention relates to sequence motifs which are specifically recognized by the Cre recombinase, a type I topoisomerase from the P1 bacteriophage. The "lox sequences" referred to herein encompass the wild type loxP recognition sequence consisting of 34 bp wherein two 13 bp palindromes (inverted repeats) are flanking an 8 bp core region and wherein the wild type loxP recombinase recognition has the sequence of SEQ ID NO.: 2. Further encompassed are mutant loxP sequences, wherein the mutant loxP sequences include sequences with not more than 8 nucleotide substitutions relative to the wild type loxP sequence of SEQ ID NO.: 2. Mutant loxP sequences with 1, 2, 3,4,5,6 or 7 nucleotide substitutions are deliberately envisaged. In accordance with the invention, the wild type loxP and the mutant loxP sequence are not compatible for recombination with each other, but are exclusively recombinable by the activity of the Cre recombinase with lox sequences with an identical sequence. Several Cre recombinases are presently known and derived via mutagenesis from the wild type bacteriophage P1 Cre recombinase in order to change features like, for example, nuclear localization or translation efficiency in mammalian cells. The skilled person is in the position to identify suitable combinations of lox sequences to be recognized by a single Cre recombinase which can be used in accordance with the present invention.

In a more preferred embodiment of the combination of DNA segments of the present invention, the recombinase is a Cre recombinase having the sequence of SEQ ID NO.: 1.

Said Cre recombinase is the wild type Cre recombinase as originally isolated from bacteriophage P1 (SEQ ID NO.: 1) and belongs to the family of DNA recombinases which catalyze site-specific recombination between two identical lox sequences.

In another more preferred embodiment of the combination of DNA segments of the invention, either the first or the second type of lox sequences has the sequence of SEQ ID NO.: 2.

Said lox sequence is the wild type loxP sequence (SEQ ID NO.: 2). It is recombined only at reduced efficiency with many mutant loxP sequences but does not recombine (below 5%, preferrably below 1% efficiency as compared to loxP x loxP recombination as assayed in vitro (Lee and Saito, Gene, 216, 55-65 (1998)) with selected mutant loxP sequences in the presence of a Cre recombinase. The recombination occurs at optimal efficiency with a second wild type loxP site having the identical sequence, i.e. SEQ ID NO.: 2, in accordance with the invention. Selected mutant lox sites in accordance with the present invention are mutant lox sites which recombine with each other in the presence of Cre recombinase with an efficiency of at least 10% relative to the efficiency of the wild type loxP x loxP recombination reaction. Advantageously, said recombination efficiency is at least 20%, 30%, 40%, 50%, 60%, 70%, 80%, 90% and most preferably at least 100%. For example, said mutant lox sequences can be lox 5171 (SEQ ID NO: 18), lox 5271 (SEQ ID NO: 19), lox 5371 (SEQ ID NO: 20), lox 5172 (SEQ ID NO: 21), lox 5272 (SEQ ID NO: 22), or lox 5372 (SEQ ID NO: 23) as published in Lee and Saito, Gene, 216, 55-65 (1998). Further examples include m2 (SEQ ID NO: 24), m3 (SEQ ID NO: 25), m7 (SEQ ID NO: 26), m11 (SEQ ID NO: 27) as published in Langer et al., Nucleic Acids Res. 30, 3067-77 (2002).

In another more preferred embodiment of the combination of DNA segments of the invention, either the first or the second type of lox sequences has the sequence of SEQ ID NO.: 3.

Said lox sequence is a mutant loxP sequence (lox2272; Araki et al., Nucleic Acids Res., 30 (19), e103 (2002)) and does not recombine with the wild type loxP sequence having the sequence of SEQ ID: NO.: 2 in the presence of Cre recombinase. The recombination only occurs with a second mutant loxP site having the identical sequence, i.e. SEQ ID NO.: 3, at an efficiency of at least 10% as compared to the recombination of wild type loxP sequences, in accordance with the invention.

In a preferred embodiment of the combination of DNA segments of the invention, the transcriptional stop element contains sequences that interfere with RNA polymerase III driven transcription.

RNA polymerase III is a polymerase which transcribes DNA to synthesize ribososmal 5S rRNA, tRNA and other small RNAs.

Transcriptional stop elements of RNA polymerase III have been first reported by Brown et Brown, (1976), J. Mol. Biol., 102, 1-14, and the signal has been characterized as four or more consecutive thymidine residues on the non-coding strand and G+C richness of the flanking DNA of the 5 S rRNA gene of frog cells. A transcriptional stop element that interferes with RNA polymerase III transcriptional activity in accordance with the invention are, for example, short repeats of at least 5 or more thymidine bases. Preferred are repeats of at least 6 thymidine bases.

In another preferred embodiment of the combination of DNA segments of the present invention, the promoters are promoters of genes transcribed by RNA polymerase III.

Genes being transcribed by RNA polymerase III are preferably so-called "housekeeping" genes the expression of which is required in all cell types and most developmental and environmental conditions. Thus, the regulation of RNA polymerase III is primarily tied to cell growth and the cell cycle and less complex than the initiation and regulation of the transcriptional process mediated by RNA polymerase II.

It is envisaged that a promoter which mediates the activity of RNA polymerase III and thus is cell-type independent and requires less co-factors is especially suitable for the expression of shRNA molecules in accordance with the invention. Such promoters are, for example, tRNA gene regulating promoters, rRNA, snRNA and miRNA gene regulating promoters.

Accordingly, in a more preferred embodiment of the combination of DNA segments of the invention, the promoters are selected from the group consisting of U6 or H1 gene promoters (Park and Kunkel, Biochem Biophys Res Commun, 214, 934-940 (1995); Myslinski, et al., Nucleic Acids Res, 29, 2502-2509 (2001)). These promoters are driven by RNA Polymerase III and allow the efficient production of short RNA molecules with defined ends and no sequence additions, since the termination signal of RNA Polymerase III consists of a stretch of thymidine residues. Both promoters are short (100 - 200 bp) and contain two sequence elements essential for transcription, the TATA box and the proximal sequence element (PSE).

In a more preferred embodiment of the combination of DNA segments of the invention, said DNA molecule containing said combination of DNA segments comprises further elements allowing for stable integration of said molecule into the genome of a non-human animal.

"Stable integration" in accordance with the present invention relates to the incorporation of a DNA sequence into the genome of a non-human mammal. This is in contrast to DNA sequences which are introduced into a cell and are only transiently transcribed and expressed extrachromosomally.

Analysis of function and expression of transfected genes may require the stable integration of the transfected DNA into the host genome. A variety of methods exists to transfect cells and said methods are well-known to the skilled person (see, for example, Sambrook, Molecular Cloning, A Laboratory Manual, Cold Spring Harbor Laboratory (1989) N.Y. and Ausubel, Current Protocols in Molecular Biology, Green Publishing Associates and Wiley Interscience, N.Y. (1889)). In the present case, it is preferred that the combination of DNA segments of the invention is stably integrated into the genome of a non-human mammal or into the genome of any eukaryotic cell except human ES cells in vitro or in vivo. The stable integration allows for the continuous and persistent expression of shRNA molecules with subsequent RNAi-mediated knockdown of the target genes and may be achieved by a variety of methods including, for example, pronuclear injection, lentiviral infection or electroporation with for example, subsequent homologous recombination-mediated integration. In this case, elements for stable integration generally consist of sequences complementary to sequences of the targeted genome. For example, a shRNA vector could be integrated into the ubiquitous active Rosa26 locus by flanking the vector with Rosa26 homology sequences and subsequent introduction into embryonic stem cells.

In a more preferred embodiment of the combination of DNA segments of the invention, said further elements allow for site-specific integration.

"Site-specific integration" as used herein relates to the stable integration of a combination of DNA segments of the invention at a specific defined genetic locus of the genome of a non-human mammal or any eukaryotic cell except human ES cells in vitro or in vivo. Preferably, the combination of DNA segments of the present invention is integrated site-specifically. Site-specific incorporation allows for controlling the function and/or activity of the integrated combination of DNA segments. The transcriptional activity of the genetic locus chosen for integration can directly influence the expression of the integrated combination of DNA segments and further the copy number of the combination of DNA segments in the genome can be limited according to the occurrence of said chosen genetic locus. The person skilled in the art is in the position to choose an appropriate genetic locus for integration of the combination of DNA segments of the invention according to his preferences regarding function and/or activity of said combination of DNA segments.

A preferred approach to integrate the combination of DNA segments of the invention stably and site-specifically is the RMCE (recombinase-mediated cassette exchange) approach. In this approach, the donor, i.e. the combination of DNA segments of the invention, and the target sequences, i.e. cassette at the genetic locus, or commonly referred to as cassettes, are each flanked by recognition sites for site-specific recombinases. Crossover events occurring on both sides of the donor and target sequences result in a clean exchange of the target cassette with the donor cassette. To ensure stability of the integrant, cassettes can be flanked by either inverted or preferably heterotypic recognition sequences, with both strategies allowing exchange between the donor and target but preventing excision of either cassette. Further this strategy ensures the integration of the donor cassette itself, rather than the backbone. Because RMCE involves the exchange of sequences rather than the simple insertion of a transgene, it provides two advantages: (a) only the sequence of the DNA sequences within the cassette, i.e. the combination of DNA segments of the invention, is integrated, in contrast to strategies that use a single recombinase recognition site leading to incorporation of the entire donor sequence; and (b) integration of sequences that do not on their own produce a phenotype is possible, thus a successful exchange can be detected simply by the loss of a marker carried by the target cassette and/or the gain of a marker carried by the donor cassette. Both of these advantages reduce the amount of extraneous sequence required for integration, thereby simplifying cloning steps and precluding any requirement for nearby transcriptional units that may influence gene expression.

In another more preferred embodiment of the combination of DNA segments of the invention, the further elements for stable and site-specific integration into the genome are sequences suitable for integration of the combination of DNA segments through recombination.

"Recombination" as used herein has been described supra and is the method preferably used for integration of the combination of DNA segments into the targeted genome. More preferred are sequences which allow for RMCE leading to the integration of the combination of DNA segments of the invention. These sequences may, for example, be encompassed in a donor cassette used for RMCE wherein said cassette comprises a pair of recombinase recognition sites flanking both a resistance gene and the combination of DNA segments of the invention.

In accordance with the above, in another more preferred embodiment of the combination of DNA segments of the invention, the site of integration is a genetic locus comprising sequences suitable for integration of the combination of DNA segments through recombination.

To allow for stable and site-specific integration of the combination of DNA segments of the invention into a genome through recombination the genetic locus comprises sequences which are identical to the sequences suitable for integration through recombination of the combination of DNA segments of the invention. Thus, the combination of DNA segments may be integrated into the genome via a recombination event. Preferred are sequences which allow for RMCE leading to the incorporation of the combination of DNA segments of the invention. These sequences may, for example, be encompassed in an acceptor cassette used for RMCE wherein said cassette comprises a pair of recombinase recognition sites flanking a resistance gene under the control of a promoter regulating the expression of said resistance gene.

In a most preferred embodiment of the combination of DNA segments of the invention, said sequences suitable for integration through recombination are recognition sites for enzymes mediating recombination events.

Said "recognition sites for enzymes mediating recombination events" have been described supra (cf. "Recombinase recognition sequences"). The recognition sites for the stable and site-specific integration of the combination of DNA segments are recognized by a different enzyme than the recombinase recognition sequences flanking the transcriptional stop element. In other words, an enzyme used for excising the transcriptional stop elements may not be able to mediate stable and site-specific integration of the combination of DNA segments of the invention. Preferably said enzyme is an integrase, such as phiC31 Integrase.

Accordingly, in a more preferred embodiment of the above embodiment of the combination of DNA segments of the invention, the enzymes mediating recombination events are DNA recombinases or integrases.

The mode of action of DNA recombinases and integrases has been described supra. Recombinases used in accordance with the present invention are, for example, FLP recombinase, that mediates recombination between identical FRT recognition sites. Integrases as used in accordance with the present invention are, for example, phage integrases that mediate unidirectional site-specific recombination between two DNA recognition sequences, the phage attachment site (attP), and the bacterial attachment site (attB). Some phage integrases require host cofactors for strand cleavage and ligation. The recognition sites are relatively short, yet long enough to be specific on a genomic scale and are thus usable in the context of large eukaryotic genomes. Preferably, the phiC31 integrase is used to mediate stable and site-specific integration of the combination of DNA segments of the invention, wherein the genetic locus comprises attP sequences, the combination of DNA segments of the invention comprises attB sequences and by the activity of phiC31 integrase are recombined.

In another more preferred embodiment of the combination of DNA segments of the invention, said DNA molecule containing said combination of DNA segments is integrated into a genetic locus having a mild or ubiquitous transcriptional activity.

A genetic locus according to the invention encompasses any point of the genome, preferably displaying transcriptional activity. As described herein, promoters can regulate the expression of genes due to their internal structure which mediates attachment and dissociation of inter alia transcription factors. Hence, the presence and structure of a promoter determines the degree of transcriptional activity of a genetic locus. The rate of expression of shRNA molecules will thus be at least partially dependent on the transcriptional activity of the genetic locus for integration. Thus, the degree of knockdown of the targeted genes can be controlled. For example, integration at a genetic locus having a ubiquitously transcriptional activity may lead to a high degree of knockdown of the targeted genes whereas a genetic locus with less transcriptional activity may lead to a lower degree of knockdown of said genes. The person skilled in the art will be in the position to choose using his general common knowledge a suitable genetic locus displaying the desired level of transcriptional activity. Further he may retrieve information on said genetic loci using well-known databases such as maintained by the National Center for Biotechnology Information (NCBI; http://www.ncbi.nlm.nih.gov/) or the EMBL-EBI and the Wellcome Trust Sanger Institute (Ensembl; http://www.ensembl.org/index.html).

In a more preferred embodiment of the combination of DNA segments of the invention, said combination of DNA segments is integrated at the Rosa26 locus or Hypoxanthin-Phosphoribosyl-Transferase (HPRT) locus. These loci are widely or ubiquitously expressed in mouse tissues and also enable the undisturbed expression of transgenic vectors inserted into these loci.

In a further embodiment the invention relates to a method of producing a transgenic non-human animal, the method comprising the steps of (a) integrating the DNA molecule containing said combination of DNA segments of the invention into the genome of a non-human animal; and of (b) crossing said animal with an animal transgenic for an expressible recombinase gene, wherein said recombinase recognizes the recombinase recognition sequences flanking the transcriptional stop element of said DNA segments.

The term "transgenic non-human animal" as used in accordance with the invention relates to an animal in which there has been a deliberate modification of its genome.

A method for the production of a transgenic non-human animal, for example, a transgenic mouse, comprises introduction of the combination of DNA segments of the invention into a germ cell, an embryonic cell, stem cell or an egg or a cell derived therefrom. Production of transgenic embryos and screening of those can be performed, e.g., as described by A. L. Joyner Ed., Gene Targeting, A Practical Approach (1993), Oxford University Press. The DNA of the embryonic membranes of embryos can be analyzed using, e.g., Southern blots with an appropriate probe. Integration of the combination of DNA segments into the genomes of non-human animals can be achieved by a variety of methods well-known to the person skilled in the art. General methods for making transgenic non-human animals are described in the art, see, for example, WO 94/24274. Such methods include, but are not limited to (a) DNA microinjection, (b) embryonic stem cell-mediated gene transfer and (c) retrovirus-mediated gene transfer.
(a) DNA microinjection involves the direct microinjection of a chosen DNA construct from any species into the pronucleus of a fertilized non-human ovum. However, the insertion of the DNA is a random process and there is a high probability that the introduced combination of DNA segments does not insert itself into site in the host genome that will permit its expression. The manipulated ovum is transferred into the oviduct of a recipient non-human female or artificially induced recipient non-human female.
(b) Embryonic stem cell-mediated gene transfer involves prior insertion of the combination of DNA segments by homologous recombination into an in vitro culture of non-human embryonic stem (ES) cells. These cells are then incorporated into a non-human embryo at the blastocyst stage of development. The result is a chimeric non-human animal. ES cell-mediated gene transfer is the preferred method for gene inactivation. It has the advantage of allowing precise targeting of defined mutations in the gene via homologous recombination.
(c) This gene transfer is mediated by means of a carrier or vector, generally a virus or a plasmid to increase the probability of expression. Retroviruses are commonly used as vectors to transfer genetic material into the cell, taking advantage of their ability to infect host cells in this way. Offspring derived from this method are chimeric as not all cells carry the retrovirus. Transmission of the transgene is possible only if the retrovirus integrates into some of the non-human germ cells.

Any of the above methods includes testing the first generation (F1) of non-human animals for the expression of the shRNA molecule directly or indirectly. Chimeras may then need to be inbred for a several more generations to obtain homozygous non-human animals.

Preferred for making transgenic non-human animals of the invention (which include homologously targeted non-human animals) are embryonic stem cells (ES cells). Murine ES cells, such as, for example, AB-1 line grown on mitotically inactive SNL76/7 cell feeder layers (McMahon and Bradley, Cell 62:1073-1085 (1990)) essentially as described (Robertson, E. J. (1987) in Teratocarcinomas and Embryonic Stem Cells: A Practical Approach. E. J. Robertson, ed. (Oxford: IRL Press), p. 71-112) or embryonic fibroblasts may be used for homologous gene targeting. Other suitable ES lines include, but are not limited to, the E14 line (Hooper et al., Nature 326:292-295 (1987)), the D3 line (Doetschman et al., J. Embryol. Exp. Morph. 87:27-45 (1985)), the CCE line (Robertson et al., Nature 323:445-448 (1986)), the AK-7 line (Zhuang et al., Cell 77:875-884 (1994)), or the IDG26.10-3 ES cells (described in the appended example).

The success of generating a mouse line from ES cells bearing the combination of DNA segments of the invention depends on the pluripotence of the ES cells (i. e., their ability, once injected into a host developing embryo, such as a blastocyst or morula, to participate in embryogenesis and contribute to the germ cells of the resulting animal). The blastocysts containing the injected ES cells are allowed to develop in the uteri of pseudopregnant non-human females and are born, e.g. as chimeric mice. The resultant transgenic mice are chimeric for cells having said combination of DNA segments or not and are backcrossed and screened for the presence of the correctly targeted combination of DNA segments by PCR or Southern blot analysis, for example, on tail biopsy DNA of offspring so as to identify transgenic mice homozygous for the combination of DNA segments.

Preferably and as outlined in the appended example, the vector comprising the combination of DNA segments of the invention as part of the donor cassette for RMCE and a recombinase expression vector are co-electroporated into ES cells carrying the acceptor cassette for RMCE. After transfection, the ES cells are selected for their ability to survive treatment with an agent for selection. ES cells having undergone successful RCME express the resistance gene contained within the donor cassette, wherein the agent for selection is chosen according to the selected resistance gene, e.g., neomycin positive ES cells will survive treatment with G418. Subsequently, single colonies are picked and maintained undifferentiated. Finally, ES cells are introduced as described above into blastocysts and mice with germline transmission are crossed homozygous for the recombinase recognition sequence hairpin allele. These homozygous mice are then crossed to a recombinase-expressing mouse strain to mediate the activation of the hairpins in the desired tissue(s), wherein said recombinase recognizes the recombinase recognition sequences flanking the transcriptional stop elements of the combination of DNA segments of the invention. Preferably, said recombinase is under the control of a tissue-specific or ubiquitous promoter.

The transgenic non-human animals may, for example, be transgenic mice, rats, hamsters, dogs, monkeys, rabbits, pigs, or cows.

In another embodiment the invention relates to a genetically engineered non-human animal transgenic for the DNA molecule containing said combination of DNA segments of the invention and an expressible recombinase gene.

Said animal is preferably produced by the method of the invention and can be used in accordance with the invention, for example but not limited to, in a method of simultaneously knocking down two genes or for identification of a combination of target genes as a potential drug target as described herein below.

In a preferred embodiment of the genetically engineered non-human animal of the invention, the recombinase is expressed under the control of a tissue-specific promoter.

The term "tissue-specific promoter" as used in accordance with the present invention relates to promoters, which are genomic DNA sequences that enable and control transcription of the gene(s) they are associated with. The concerted regulation of gene expression is especially important in multicellular organisms. Thus, promoters are involved in a complex coordination of transcription under all conceivable spatiotemporal-conditional circumstances. This is achieved by their internal structure, consisting of arrays of individual protein (e.g., transcription factors) binding sites, that form a hierarchical structure of modules, i.e. functionally important and transferable combinations. Regarding tissue-specificity it is well-known in the art that gene expression varies at different stages in the development of cells (e.g. senescence, differentiation) and varies from cell type to cell type. Cell types have - due to differential expression - characteristic profiles of the genes expressed. The characteristic array of expressed genes can be used for classifying a cell, i.e. what kind of cell, what developmental stage, normal or diseased. A tissue-specific promoter according to the invention is a promoter which can be used to classify cells, i.e. differentiate cells, and is preferably exclusively active in a single class of cells relative to other cells or cell classes. The person skilled in the art is aware of several tissue-specific promoters and further tissue-specific promoters can be found according to methods as described, for example, in Chen et al., (2006), Nucleic Acids Research, 34, database issue D104-D107. Further, genetically engineered non-human animals transgenic for a recombinase under the control of a tissue-specific promoter can be obtained from several well-known facilities such as, for example, The Jackson Laboratory (Bar Harbor, ME, USA), B & K Universal Limited (Hull, England), Charles River Laboratories, Inc. (Wilmington, MA, USA), Harlan (Indianapolis, IN, USA) or Taconic Farms, Inc. (New York, NY, USA).

In a preferred embodiment of the method or the genetically engineered non-human animal of the invention, the non-human animal is a rodent.

Rodents, for example, rats and mice, have been shown to be suitable as research tool (e.g. disease model) for several reasons. For example, mice adapt well to laboratory housing and can be housed socially or individually. Significant numbers can be housed in relatively little space because of their small body size. Further, they possess a surprising genetic similarity to humans - approximately 85% similarity on a bp to bp basis. Furthermore, it has been shown that 90 % of genes linked to diseases are the same in mice as in humans. These features - the similar gene sequence, genetic content and arrangement of genes - combined with a rapid rate of reproduction, make rodents, preferably mice, the non-human animal of choice for genetic manipulation as envisaged and described herein.

Accordingly, in a more preferred embodiment of the method or the genetically engineered non-human animal of the invention, the rodent is a mouse.

Further, in an embodiment the present invention relates to a eukaryotic cell genetically engineered with the combination of DNA segments of the invention.

The cell can be any eukaryotic cell wherein the combination of DNA segments has been preferably introduced into the genome of said cell via recombination or is maintained extrachromosomally as part of vectors and upon excision of the transcriptional stop elements through the activity of a recombinase as described herein above, shRNA molecules are expressed which knock down the expression of the target genes through RNAi. Also preferred is that the combination of DNA segments used for engineering said eukaryotic cell is contained in the same DNA molecule.

The term "eukaryotic" is meant to include yeast cells, cells of higher plants, insect cells and preferably mammalian cells, such as, for example, a non-human ES cell as described herein. A combination of DNA segments of the invention can be used to transform or transfect said cell using any of the techniques commonly known to those of ordinary skill in the art and, for example, described in Sambrook, Molecular Cloning, A Laboratory Manual, Cold Spring Harbor Laboratory (1989) N.Y. and Ausubel, Current Protocols in Molecular Biology, Green Publishing Associates and Wiley Interscience, N.Y. (1889).

In another embodiment the invention relates to a method of simultaneously knocking down two genes in a eukaryotic cell comprising the steps of: (a) introducing the combination of DNA segments of the invention into a cell; (b) excising the transcription stop elements of (ab) and (bb) through the activity of a recombinase.

The combination of DNA segments of the invention may be introduced into a cell according to methods well-known to the person skilled in the art and described herein above and below. The mode of action of recombinases has also been described herein and is well-known in the art. Also preferred is that the combination of DNA segments used for knocking down two genes in said eukaryotic cell is contained in the same DNA molecule.

In a preferred embodiment of the cell of the invention or the above method of the invention, the recombinase is expressed in said cell.

Preferably, the functional recombinase is endogenously expressed in said cell or the gene encoding said recombinase has been introduced into the cell according to methods well-known in the art and maintained extrachromosomally, for example, on a plasmid or other vector, or stably integrated into the genome and can thus be expressed.

In a more preferred embodiment of the above cell or the method of invention, the recombinase is expressed under the control of a tissue-specific promoter.

As described herein above, tissue-specific promoters are confined in exerting their activity in a specific cell class. Thus, the use of a tissue-specific promoter as promoter for regulating the expression of the recombinase will lead to the expression of said shRNA molecules and subsequent knockdown of the target genes only in cells in which said promoter is active. This conditional knockdown is advantageous in a variety of therapeutic or experimental settings, for example, the study of tissue-specific diseases or the effect of a disease on a specific tissue or compartment of the body, generation of nonlethal mutations in non-human animal embryos, as outlined throughout the specification.

In a preferred embodiment of the method of the invention, the recombinase is exogenously introduced into the cell.

The recombinase intended to excise the transcription stop elements in order to allow for the expression of shRNA molecules may be added to the cell and, upon uptake, will exert activity within the cell. The person skilled in the art is aware of conditions generally suitable for uptake of proteins, including enzymes such as recombinases, into cells and methods to enhance said uptake as regards rate and amount wherein said enhancement may include artificially modifying proteins (see, for example, Patsch et Edenhofer, (2007), Handb Exp. Pharmacol., 178, 203-232). Furthermore, (s)he is also aware of cell lines naturally exhibiting the capacity of increased protein uptake relative to other cells. Such cells are, for example, cells like mucosal cells or intestinal cells. A number of mechanisms exist for the passage of proteins across the plasma membrane, including passive diffusion, facilitated diffusion, and active transport systems. Passive diffusion of proteins through the bilayer lipid structure of the plasma membrane is a function of the size, lipid solubility, and charge of the protein molecule. A further uptake mechanism is endocytosis. Endocytosis is a process whereby cells absorb material from the outside by engulfing it with their cell membrane. Endocytosis works with macromolecules or particulate matter beyond a certain size threshold.

In a further embodiment, the invention relates to a method of identifying a combination of two target genes as a potential drug target comprising the steps of: (a) determining different expression or activity of nucleic acid molecules or proteins in a cell exhibiting characteristics associated with a disease and in a normal cell; (b) knockdown of two genes according to the method of the invention in said cell exhibiting characteristics associated with a disease; and (c) determining the effect of the knockdown on said cell exhibiting characteristics associated with a disease; wherein a change in said disease characteristics is indicative that said combination of two target genes is a potential drug target.

The identification of drug targets aims at providing information on how to treat diseases. Diseases as used herein encompass without limitation any disease being triggered, exacerbated by and/or related in any way to the presence of endogenous factors (e.g. genetic predisposition) and/or ambient factors (e.g. bacteria, viruses). Said diseases may, for example, be associated with discomfort, dysfunction, distress, injury, disability, syndromes, infections, changes in behaviour, atypical variations in structure and function. While many diseases are biological processes with observable alterations of organ function or structure, others may primarily or exclusively involve alterations of behaviour.

Different expression or activity of nucleic acid molecules or proteins can be determined by methods well-known in the art (see, for example, "Molecular Cloning: A Laboratory Manual" by Sambrook et al. (Cold Spring Harbour Laboratory Press) or "Current Protocols in Molecular Biology" (Ausubel et al., Wiley and Sons, Inc); "Analysing Gene Expression, A Handbook of Methods: Possibilities and Pitfalls" by Stefan Lorkowski, Paul M. Cullen (eds.); Wiley-VCH, Weinheim. The methods preferably are highly sensitive and provide reproducible results. Examples of such methods are flow-cytometry, immunoassays (e.g., ELISAs), affinity mass spectrometry, preferably DNA- and protein-microarrays. Further, methods based on the detection of mRNA level alteration, which are in particular, methods based upon the polymerase chain reaction (real-time PCR) and related amplification technologies, such as NASBA and other isothermal amplification technologies, may be used.

As described above, characteristics of diseased cells can be any symptom known to be associated with a disease, as, for example, aberrant structure and/or function of the cell and/or as well as changes in gene expression commonly associated with a disease. Preferably the disease characteristics are detectable with any of the above methods regarding detection of different expression or activity of nucleic acid molecules or proteins. This is, for example, the case in cancer cells of various different cancers, which can be classified according to inter alia their gene expression profile. The person skilled in the art is well aware of further disease characteristics or may retrieve information from databases providing, for example, information on DNA- and/or protein arrays.

Determining the effect of a knockdown may be detection of any change of the disease characteristics, wherein disease characteristics can be a combination of several symptoms or only one symptom. Suitable methods for detecting the effect depend on the nature of the disease characteristic. For example, any of the above recited methods for determining the expression or activity of nucleic acid molecules or proteins can be used when a disease characteristic is the aberrant expression or activity of a nucleic acid molecule or protein relative to a control, as, for example, in cancer cells. Preferably, methods suitable to provide measurable and reproducible results may be used to detect the effect being a change of the disease characteristics. A change encompasses a decrease or increase of a factor which qualitatively and/or quantitatively describes said disease characteristics, and preferably involves the disappearance of said disease characteristics.

The above applies mutatis mutandis for the embodiments supra and infra.

In a preferred embodiment of the method of the invention, at least one of the genes is known to be associated with said disease.

Genes associated with a disease(s) are well-known to the person skilled in the art. Further, annotated records for known sequence variations in the human genome can be found at the Human Genome Variation database (HGVbase, http://hgvbase.cgb.ki.se/) and may provide further insight in genes associated with a disease. Said genes may be genes which are, for example, indicative of, causative for and/or contributory to a disease. The second gene can be any other gene, preferably a gene identified as being differently expressed or having a different activity according to step (a). Alternatively, said second gene may be located within the linkage disequilibrium (LD) block (as generated during haplotype analysis, e.g. for genomic screening for diseases) of the gene known to be associated with a disease, wherein the LD block is a DNA segment within which markers are in significant linkage disequilibrium with each other, which implies that there is low recombination activity within that block.

In another preferred embodiment of the method of the invention, the cell exhibiting characteristics associated with a disease is to be obtained from a patient.

Methods for obtaining samples containing diseased cells are well-known in the art and encompass, for example, isolation of blood cells, cells in the mucus, bile, stool, saliva and other easily accessible cells of the patient as well as cells obtained through surgery as part of tissue samples or by-products of surgery depending on the disease type. A cell obtained accordingly can be cultured according to methods well-known in the art as a primary cell line or modified, for example, immortalized to be used as a permanent cell line. Protocols for the isolation of different cell types, their culture conditions, and for the evaluation of the degree of differentiation of a primary cell culture are known in the art. In the case of tissue samples, mononuclear cells can be obtained, for example, by enzymatic digestion with enzymes such as collagenase, trypsin or pronase or any other enzyme breaking down the extracellular matrix. Another strategy involves placing the tissue sample in growth media, and cells that grow are available for tissue culture, the method being termed explant culture. A variety of methods and kits to isolate specific cell-types are well-known in the art and may be obtained, for example, from Qiagen or Miltenyibiotech. Immortalizing a primary cell line may be achieved by random mutation or deliberate modification, such as, for example, artificial expression of the telomerase gene. Other methods are well-known to the person skilled in the art. There are numerous well established and characterized cell lines representative of particular cell types, both normal and diseased. Said cell lines may be obtained, for example, at the American Type Culture Collection (ATCC, www.atcc.org) or Deutsche Sammlung von Mikroorganismen und Zellkultur (DSMZ, www.dsmz.de).

Culture conditions vary from cell-type to cell-type and moreover, can result in different phenotypes being expressed for a particular cell-type. Generally, cells are grown and maintained at an appropriate temperature and gas mixture, i.e. typically 37°Celsius, 5% CO₂, in growth media (a) as irrigating, transporting and diluting fluid while maintaining intra- and extra-cellular osmotic balance, (b) that provides cells with water and certain bulk inorganic ions essential for normal cell metabolism, (c) which - combined with a carbohydrate, such as glucose - provides the principle energy source for cell metabolism and (d) which provides a buffering system to maintain the medium within physiologic pH range, i.e. cells are kept viable. The recipe of growth media varies greatly depending on cell-type and contains, for example and without limitation, growth factors, nutrient components, glucose, buffers to maintain pH and antifungizides and -biotics. Methods for culturing and maintaining cells in culture are well-known in the art and described, for example, in "Practical Cell Culture Techniques", Boulton et Baker (eds), Humana Press (1992), ISBN 0896032140; "Human Cell Culture Protocols", Gareth E. Jones, Humana Press (1996), ISBN 089603335X; growth media and other cell culture related material as well as instructions and methods for successful culturing of cells can, for example, be obtained at Sigma-Aldrich.

In a preferred embodiment of the above method of the invention, steps (a) to (c) are performed in a mouse.

As described herein above, mice have proven to be advantageous as a disease model for a variety of human diseases. Hence a variety of mouse lines and protocols have been developed to generate disease models in order to elucidate aspects of a target disease. Preferably, for determination of different expression or activity in step (a) a control mouse is used, i.e. a mouse without knockdown of two genes but manipulated to show symptoms of a disease. Said control mouse advantageously is a mouse only transgenic for an expressible recombinase gene. Further preferred, in step (b) the cell used is part of the transgenic mouse of the invention manipulated to show symptoms of a disease and determination of the effect in step (c) is performed in cells obtained from the transgenic mouse of the present invention relative to cells obtained form the control mouse of step (a). In other words, the transgenic mouse of the invention is used as a disease model and the effect of the knockdown of the two genes on the disease characteristics is compared to the disease characteristics of the control mouse also used as a disease model, wherein the disease models are the same. The skilled person will be able to obtain diseased cells of a such modified animals and determine different expression or activity of nucleic acid molecules or proteins according to any of the methods described herein and well-known to person skilled in the art.

In a preferred embodiment of the combination of DNA segments, the cell or the method of the invention, said eukaryotic cell is selected from the group consisting of non-human embryonic stem cells, cells contained in a tissue sample and a cell contained in a transgenic non-human mammal.

As described herein-above, non-human embryonic stem cells are essential to produce non-human transgenic animals of the invention. Further, the combination of DNA segments may be introduced into a eukaryotic cell being part of a tissue sample or of a transgenic non-human animal.

The present invention furthermore provides a pharmaceutical composition comprising the combination of DNA segments of the invention.

Finally, the present invention relates to the use of the combination of DNA segments of the invention for the preparation of a composition for gene therapy. Also provided is the combination of DNA segments of the invention for gene therapy.

The use of the combination of DNA segments of the invention encoding functional and expressible shRNA molecules is intended for the preparation of a composition for treating, preventing and/or delaying a disorder diagnosed by the method of the invention or known in the art. Gene therapy, which is based on introducing therapeutic DNA constructs into cells by *ex vivo* or *in vivo* techniques is one of the most important applications of gene transfer. Suitable vectors and methods for *in vitro* or *in vivo* gene therapy are described in the literature and are known to the person skilled in the art; see, e.g., Giordano, Nature Medicine 2 (1996), 534-539; Schaper, Circ. Res. 79 (1996), 911-919; Anderson, Science 256 (1992), 808-813; Isner, Lancet 348 (1996), 370-374; Muhlhauser, Circ. Res. 77 (1995), 1077-1086; Wang, Nature Medicine 2 (1996), 714-716; WO 94/29469; WO 97/00957 or Schaper, Current Opinion in Biotechnology 7 (1996), 653-640, and references cited therein. The DNA construct may be designed for direct introduction or for introduction via liposomes, or viral vectors (e.g. adenoviral, retroviral) into the cell and as described above. Preferably, said cell is a non-human germ line cell, non-human embryonic cell, or non-human egg cell or derived therefrom, most preferably said cell is a stem cell, except a human ES cell. Suitable gene delivery systems that can be employed in accordance with the invention may include liposomes, receptor-mediated delivery systems, naked DNA, and viral vectors such as herpes viruses, retroviruses, adenoviruses, and adeno-associated viruses, among others known in the art or described herein. Delivery of nucleic acids to a specific site in the body for gene therapy may also be accomplished using a biolistic delivery system, such as described by Williams (Proc. Natl. Acad. Sci. USA 88 (1991), 2726-2729). Standard methods for transfecting cells are well known to those skilled in the art of molecular biology, see, e.g. WO 94/29469; see also supra. Gene therapy may be carried out by directly administering the combination of DNA segments or vector of the invention ex vivo and infusing cells into the patient.

The figures show:

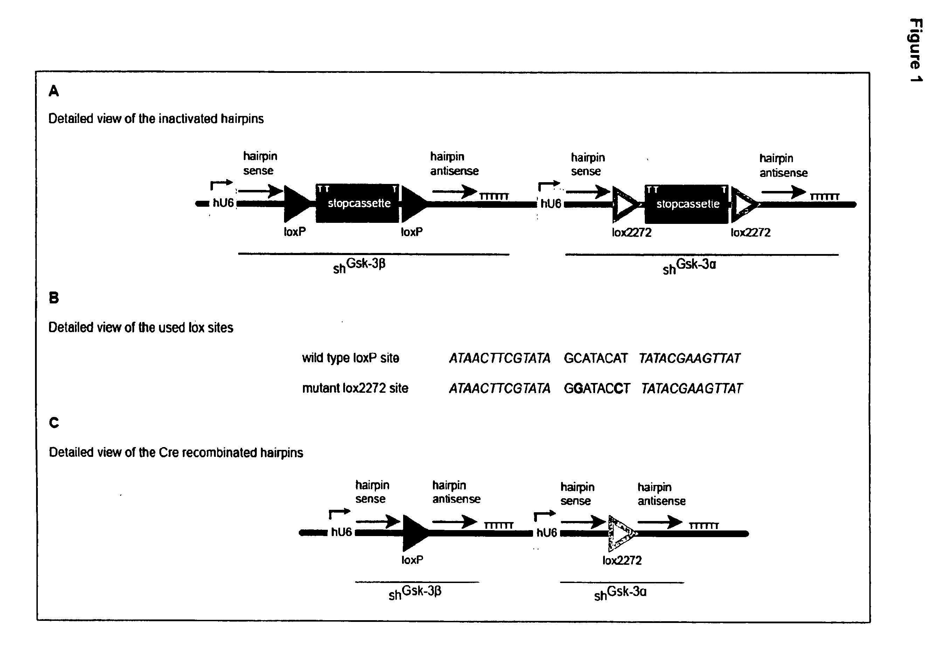

**Figure 1****:** Conditional short hairpin constructs
   The short hairpins are driven by a human U6 promoter and are interrupted in their loop region by a stop cassette. The stop cassette consists of a 800 base pairs spacer, flanked by Cre recombinase recognition sites, so called lox sites. Additionally, there are several stop signals situated in the stop cassette to disable the transcription completely (A). The lox sites of the short hairpins differ in two base pairs. The inverted repeats are marked in italic, while the mutated base pairs in the lox2272 site are marked in red (B). During the Cre recombinase mediated deletion the stop cassettes are cut out, leaving one of the lox sites behind. The remaining lox sites do not interfere with the activity of the short hairpins (C).

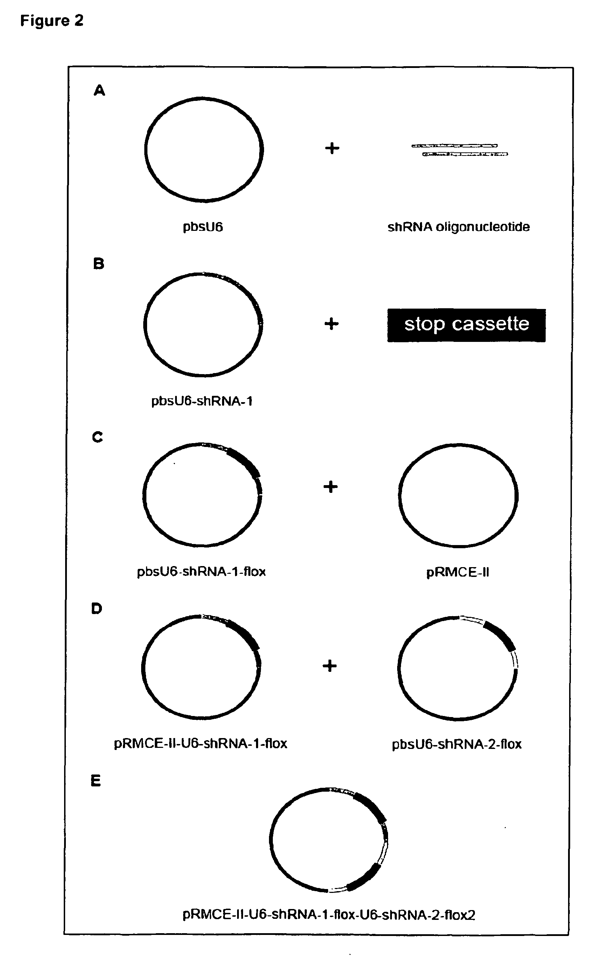

**Figure 2****:** Cloning Strategy
   First, the short hairpin oligo-nucleotides are cloned behind a U6 promoter (A). In the next step, the stop cassette is integrated into the loop region of the hairpin (B). The conditional hairpin is transferred into an exchange vector, containing a resistance gene and recognition sites for the C31 integrase (C). The second conditional hairpin is cloned into the exchange vector behind the first construct (D). Finally, both conditional hairpin constructs, each with its independent U6 promoter, are situated in a row in the exchange vector for recombinase mediated cassette exchange (E).

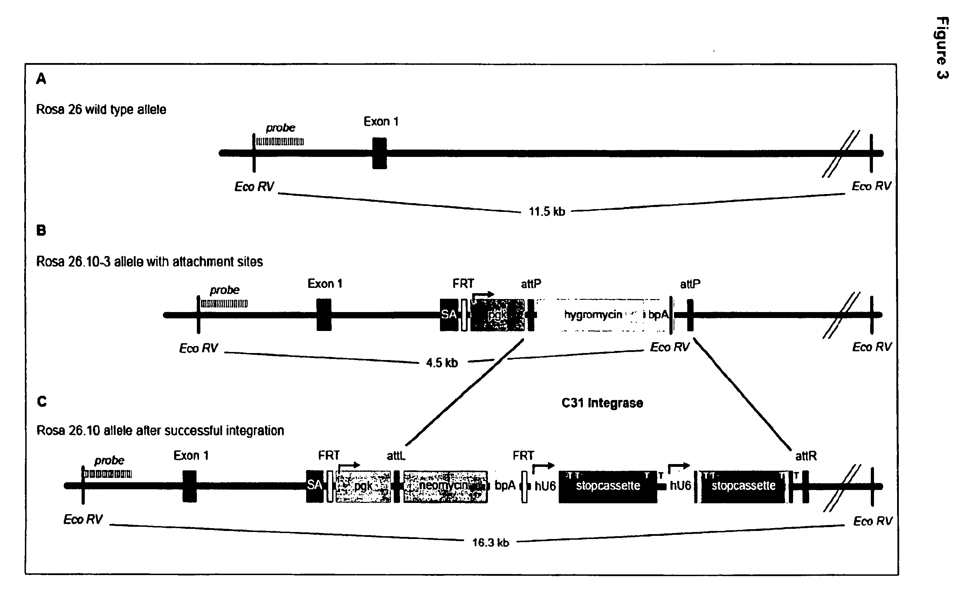

**Figure 3****:** Generation of recombinant ES cells
   The hairpin expression cassettes are integrated in the *Rosa26* locus in the mouse genome (A). The wild type locus is modified with an acceptor cassette that consists of a pair of recognition sites of the C31 Integrase, flanking a hygromycin resistance gene, driven by a pgk promoter (B). Cotransfection of the hairpin expression cassettes and a C31 Integrase expression vector into ES cells leads to the stable integration of the hairpins into the genome (C).
**Figure 4****:** Functional knockdown of Gsk-3α and Gsk-3β
   As control wild type (wt) mice or heterozygous floxed (+/flox) mice were used. Mutant mice are heterozygous for the active hairpin (+/Δ) and carry a copy of the Nestin-controlled Cre recombinase.
**Figure 5****:** Structure of shRNA molecules
   Schematic drawing of elements comprised in one of the DNA segments making up the combination according to the invention before and after a recombination event mediated by Cre recombinase and the resulting RNA transcripts.

The example illustrates the invention:

### Example

Glycogen synthase kinase-3 (Gsk-3) is a serine-threonine kinase and was first identified as a consequence of its phosphorylation activity toward glycogen synthase, the rate limiting enzyme of glycogen metabolism. In mammals, there are two isoforms called glycogen synthase kinase 3 alpha and beta (Gsk-3α, Gsk-3β). The two genes share 85% overall sequence identity and 93% in the catalytic domain (Ali et al., Chem Rev; 101:2527-2540 (2001)). Their discrete functions are not yet elucidated, but it is known that they share some. To investigate the role of the two kinases it is necessary to eliminate them both in the same tissues and at the same time. The invention is appropriate for this question and this example shows its application in a mouse model.

### A. Construction of conditional double short hairpin vectors

To gain control over the expression of the two short hairpins, it is necessary to insert a transcriptional stop element. Therefore, the loop region of the hairpins was chosen (Fig.1. **A**). The stop element consists of an 800 base pairs long spacer, containing several transcriptional stops. It is flanked by Cre recombinase recognition sites, which allow the deletion of the stop cassette by Cre recombinase. After the excision, one of the lox sites is left behind (Fig.1. C). It is possible to use different incompatible lox sites, to ensure the independent control over both hairpins. The wild type loxP site and the mutated lox2272 site differ in two base pairs (Fig.1. **B**).

The oligo-nucleotides, with the selected short hairpin sequences for Gsk-3α (SEQ ID NOs.: 4 (sense) and 5 (antisense)) and Gsk-3β (SEQ ID NOs.: 6 (sense) and 7 (antisense)), were cloned into a pbsU6 vector (SEQ ID NO.: 8). First, the oligo-nucleotides were annealed and subsequently ligated into the pbsU6 vector, digested with BseRI and BamHI (Fig.2. **A**). The stop cassettes were integrated into the predesigned HindIII site in the loop sequence of the shRNAs (Fig.2. **B**). The stop cassette flanked by the wild type loxP sites (SEQ ID NO.: 9) was integrated into the pbsU6 vector with the shRNA against Gsk-3β (SEQ ID NO.: 12; pbsU6-shGsk-3β-flox) and the stop cassette flanked by the mutated lox2272 sites (SEQ ID NO.: 10) was integrated into the pbsU6 vector containing the shRNA against Gsk-3α (SEQ ID NO.: 11; pbsU6-shGsk-3α-flox2). To generate mice, the vector was inserted into ES cells via recombinase mediated cassette exchange (Hitz, et al., Nucleic Acids Res, 35, e90 (2007)). Therefore, the pbsU6 vectors had to be cloned into an exchange vector (SEQ ID NO.: 13: pRMCE-II) containing recognition sites (attP sites) for integrase of the phage C31. First, the pbsU6-shGsk-3β-flox vector was digested with AsiSI and Sfil to cut out the U6 promotor and the shRNA including the stop cassette. The fragment was cloned into the exchange vector, using the same restriction sites (SEQ ID NO.: 14; pRMCE-II-U6-shGsk-3β-flox, Fig.2. **C**). The pbsU6-shGsk-3α-flox2 vector was digested with Xbal and the fragment was inserted into the pRMCE-II-U6-shGsk-3β-flox vector via the Spel restriction site (SEQ ID NO.: 15: pRMCE-II-U6-shGsk-3β-flox-U6-shGsk-3α-flox2, Fig.2. **D**). Thus, the Gsk-3α shRNA is situated downstream of the Gsk-3β shRNA (Fig.2. **E**).

Before transfecting the vector stably into ES cells, the efficiency of the shRNA vectors can be tested in transient transfections and subsequent analysis of the protein reduction in western blot.

### B. Generation and genotyping of recombinant ES cells

The tested vector was electroporated into IDG26.10-3 acceptor ES cells together with a C31 integrase expression vector (SEQ ID NO.: 16: pCAG-C31Int-bpA). The acceptor ES cells contain a modified *Rosa26* allele that consists of a pgk promotor and a hygromycin resistance gene, flanked by recognition sites (attB sites) for the C31 integrase (Fig.3. **B**) (Hitz, et al., Nucleic Acids Res, 35, e90 (2007)). C31 integrase exchanges the sequence in the genome between the attB sites with the attP flanked sequence of the introduced vector. So, the neomycin resistance and the U6 promoter with the shRNA were integrated into the *Rosa26* locus (Fig.3. **C**). The ES cells were kept on MMC treated G418 resistant embryonic fibroblasts (feeder cells). Two days after the transfection, the ES cells were selected with G418 (140µg/ml) for six days to identify recombinant clones. After this time round colonies with sharp borders were picked, separated and expanded. During this time, it is essential to prevent the differentiation of the ES cells.

To analyse the genotype of the clones, the genomic DNA of some ES cells from every expanded clone was extracted. Positive clones were analysed by PCR and Southern Blot. The integrated construct can be amplified by a primer in the pgk promoter (5'-CAC GCT TCA AAA GCG CAC GTC TG-3'; SEQ ID NO.: 28) and a reverse primer in the neomycin resistance (5'-GTT GTG CCC AGT CAT AGC CGA ATA G-3'; SEQ ID NO.: 29), giving a product of 280 base pairs at 65°C. The feeder cells, which harbour a pgk-neo resistance gene, give rise to an additional band of 160 base pairs. ES cell clones that did not undergo a complete recombination event (partial recombination, random integration, mixed clones) contain the hygromycin gene, which can be identified by a PCR, using the primers Hyg-1 (5'-GAA GAA TCT CGT GCT TTC AGC TTC GAT G -3'; SEQ ID NO.: 30) and Hyg-2 (5'-AAT GAC CGC TGT TAT GCG GCC ATT G -3'; SEQ ID NO.: 31) at a temperature of 65°C. The product of this PCR has 550 base pairs. The *Rosa26* wild type allele can be identified with the primers Rosa-5' (5'-CGT GTT CGT GCA AGT TGA GT-3'; SEQ ID NO.: 32) and Rosa-3' (5'-ACT CCC GCC CAT CTT CTA G-3'; SEQ ID NO.: 33), giving a product of 536 base pairs at 57°C. To identify the genotype of the clones via Southern Blot, the genomic DNA was digested with the restriction enzyme EcoRV. The DNA was detected with a 448 base pairs long *Rosa26* specific probe, situated upstream of the first exon and amplified with the primer pair 5'-aaggatactggggcatacg-3' (SEQ ID NO.: 34) and 5'-cttctcagctacctttacacacc-3' (SEQ ID NO.: 35). Recombined clones display the 11.5 kB *Rosa26* wild type fragment and a 16.3 kB fragment, which derives from the correct recombined locus. An 11.4 kB fragment is detectable in case of a partial recombination and without recombination the original 4.5 kB fragment of the hygromycin-positive acceptor cells can be detected. Later, the generated mice can be genotyped by the pgk-neo PCR or by Southern Blot.

### C. In vitro activation of the double hairpins in ES cells

One of the confirmed recombinant ES cell clones was expanded and transfected with a Cre recombinase expression vector (SEQ ID NO.: 17: pCAG-Cre-bpA). The transfected cells were distributed in a very low concentration of 1,000 cells per 10 cm plate. After six days the colonies were picked, separated and expanded.

To analyse the genotype of the clones, the genomic DNA of some ES cells from every expanded clone was extracted. The clones were analysed via Southern blot, using the *Rosa26* specific probe. In contrast to the protocol in section B. the genomic DNA is digested with BamHI. Cre recombinase mediated cleavage of the stop cassettes results in a 9.0 kB fragment, while the undeleted, floxed allele gives rise to a 5.4 kB fragment. A 5.8 kB fragment derives from the *Rosa26* wild type allele.

### D. Breeding and probe preparation of double knockdown mice

After generating mice out of recombinant ES cell clones and germline transmission of the mutation, the mice were crossed homozygous for the floxed hairpin allele. These homozygous mice were crossed to a Cre recombinase expressing mouse strain to mediate the activation of the hairpins in the desired tissue(s). We used a mouse strain expressing Cre recombinase under the control of the rat Nestin (Nes) promotor and enhancer (Tronche et al., Nat Genet; 23:99-103 (1999)). The Nestin-cre mice express Cre recombinase in neuronal and glia cell precursors and show a strong expression of Cre recombinase in the central and peripheral nervous system. The mutant mice are heterozygous for the hairpin and Cre recombinase. At the age of three weeks, the pups were separated from their parents and genotyped. At the age of five weeks, a wild type (wt), a heterozygous floxed (+/flox), and a mutant mouse (+/Δ) were suffocated by CO₂, decapitated and the brains were rapidly dissected on ice. The brains were bisected sagittally and one half was frozen immediately on dry ice, used later for RNA extraction or storage in liquid nitrogen. The second half was stored on ice or at -20°C until protein preparation.

### E. Immunoblotting and Quantification via Northern Blotting

For protein extraction, the tissue/ES cells were homogenized in RIPA buffer (50 mM Tris-HCl pH 7.4, 1 % NP-40, 0.25% sodiumdesoxycholat, 150 mM NaCl, 1 mM EDTA, protease inhibitor), sonificated, and centrifuged. Of each sample 10 and 20 µg protein were run on a 10% Tris-HCl gel (Biorad) and blotted on a PVDF membrane (Pall). After blocking with 4% skim milk the membrane was incubated with the first antibody (1 hour), washed with TBS-T, incubated with the second horseradish-peroxidase-conjugated antibody (1 hour) and washed with TBST. The detection reaction was initiated with ECL detection reagents (Amersham) and the membrane was exposed to Hyperfilm (Amersham). The antibodies used for Western blotting were anti-β-Actin (AC-15, #ab6276, Abcam, 1:100,000), anti-GSK-3α/β (#KAM-ST002, Stressgen, 1:10,000), anti-mouse (Dianova, 1:1,000).

The RNA was extracted from the tissue with TriReagent (Sigma) according to the manufacturer's protocol and solved in 100µl Sodiumcitrate (1mM, pH 6.4). The OD was measured and 20 µg per sample were loaded to a 1% Agarose gel. Afterwards, the RNA was transferred to a nylon membrane (Amersham) and hybridised with a gene specific probe. The radioactive labelled probe was detected with a Kodak BioMax MS film for several hours or overnight at -80°C, depending on the signal intensity. For quantification of band intensities the membrane was exposed to an Imaging Plate, from which signals were detected and scanned with the FLA-3000 imaging analyzer. After the first probe (Gsk-3α), the membrane was stripped and treated subsequently with the second gene specific probe (Gsk-3β), and at last with the loading control β-Actin.

### F. Results

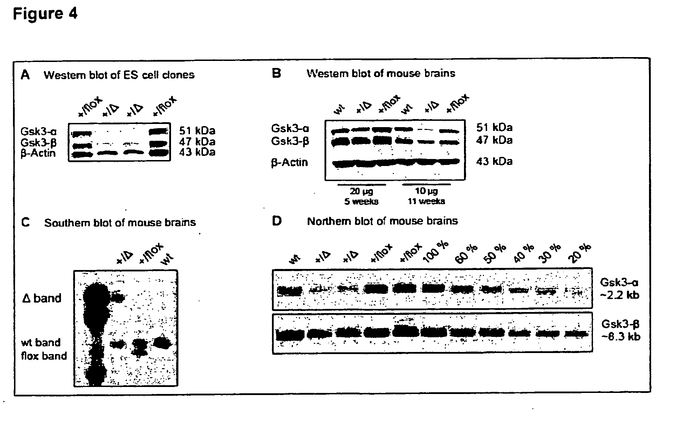

The conditional short hairpins, directed against Gsk-3α and Gsk-3β, are integrated in one step, at the same time, in direct neighbourhood, into the genome of mouse ES cells. Already *in vitro* we could achieve the proof-of-principle for this technique. We performed the Cre recombinase mediated deletion of the stop cassettes in ES cell culture by transfecting them with a Cre recombinase expression vector. A Western blot of the protein shows a clear and strong reduction of both Gsk-3 isoforms in the recombined clones (Fig.4. **A**).

Additionally, we could prove the technique *in vivo.* We crossed the generated mice with mice expressing Cre recombinase in neuronal tissues. The brains of mutant mice (+/Δ) were used for Western blots. In Fig.4. **B**, brain protein of six animals was applied with different protein amounts. The reduction in protein levels of both Gsk-3 isoforms is obvious in the +/Δ animals. To prove the total deletion of the stop cassettes, we performed a Southern blot. Fig.4. **C** shows the complete deletion of the stop cassettes. The band of the floxed allele disappeared completely in the +/Δ animal. Instead, a comparably strong band for the deleted allele appeared. Besides, we measured the knockdown efficiency by Northern blotting. Again, on mRNA level we could detect a clear reduction in the +/Δ animals. For Gsk-3α the reduction is around 60%, for Gsk-3β we determined a reduction of 50% (Fig. 4. **D**).

## Claims

1. A combination of DNA segments comprising:
(a) a first segment comprising in 5' to 3' or 3' to 5' order:
(aa) a promoter;
(ab) a first DNA sequence comprising:
(i) a DNA sequence giving rise upon transcription to the sense strand of an shRNA molecule;
(ii) a transcriptional stop element which is flanked by a first type of recombinase recognition sequences; and
(iii) a DNA sequence giving rise upon transcription to the antisense strand of an shRNA molecule;
(b) a second segment comprising in 5' to 3' or 3' to 5' order:
(ba) a promoter;
(bb) a second DNA sequence comprising:
(i) a DNA sequence giving rise upon transcription to the sense strand of an shRNA molecule;
(ii) a transcriptional stop element which is flanked by a second type of recombinase recognition sequences; and
(iii) a DNA sequence giving rise upon transcription to the
antisense strand of an shRNA molecule;
wherein
(i) said first type of recombinase recognition sequences are recognized and recombined by a recombinase but not recombined with said second type of recombinase recognition sequences;
(ii) said second type of recombinase recognition sequences are recognized and recombined by the recombinase of (i) but not recombined with said first type of recombinase recognition sequences; and
(iii) said DNA sequence of (ab) and (bb) is expressed under the control of said promoters of (aa) and (ba) upon removal of said transcriptional stop elements of (ab) and (bb) by the activity of a recombinase, resulting in transcription of said shRNA molecule in a cell.

2. The combination of DNA segments of claim 1, wherein said segments are contained in the same DNA molecule.

3. The combination of claim 1 or 2, wherein said combination of DNA segments is part of a vector.

4. The combination of DNA segments of any one of claims 1 to 3, wherein said recombinase recognition sequences are lox sequences encompassing a wild type loxP sequence and a mutant loxP sequence.

5. The combination of DNA segments of claim 4, wherein
(a) said recombinase is a Cre recombinase having the sequence of SEQ ID NO.: 1; or/and
(b) wherein either the first or the second type of loxP sequences has the sequence of SEQ ID NO.: 2; or/and
(c) wherein either the first or the second type of loxP sequences has the sequence of SEQ ID NO.: 3.

6. The combination of DNA segments of any one of claims 2 to 5, wherein the transcriptional stop element contains sequences that interfere with RNA polymerase III driven transcription.

7. The combination of DNA segments of any one of claims 1 to 6, wherein the promoters are promoters of genes transcribed by RNA polymerase III.

8. The combination of DNA segments of any one of claims 2 to 7, wherein said DNA molecule containing said combination of DNA segments comprises further elements allowing for stable integration of said molecule into the genome of a non-human animal.

9. A genetically engineered non-human animal transgenic for the DNA molecule containing said combination of DNA segments of any one of claims 2 to 8 and an expressible recombinase gene.

10. An isolated eukaryotic cell genetically engineered with the combination of DNA segments of any one of claims 1 to 8.

11. An in vitro method of simultaneously knocking down two genes in a eukaryotic cell comprising the steps of:
(a) introducing the combination of DNA segments of any one of claims 1 to 8 into a cell;
(b) excising the transcription stop elements of (ab) and (bb) through the activity of a recombinase.

12. An in vitro method of identifying a combination of two target genes as a potential drug target comprising the steps of:
(a) determining different expression or activity of nucleic acid molecules or proteins in a cell exhibiting characteristics associated with a disease and in a normal cell;
(b) knockdown of two genes according to the method of claim 11 in said cell exhibiting characteristics associated with a disease; and
(c) determining the effect of the knockdown on said cell exhibiting characteristics associated with a disease; wherein a change in said disease characteristics is indicative that said combination of two target genes is a potential drug target.

13. A pharmaceutical composition comprising the combination of DNA segments of any one of claims 1 to 8.

14. The combination of DNA segments of any one of claims 1 to 8 for gene therapy.

## Patentansprüche

1. Kombination von DNA-Segmenten umfassend:
(a) ein erstes Segment umfassend in 5' nach 3'- oder 3' nach 5'-Richtung:
(aa) einen Promotor;
(ab) eine erste DNA-Sequenz umfassend:
(i) eine DNA-Sequenz, die nach Transkription den Sinnstrang eines shRNA-Moleküls ergibt;
(ii) ein transkriptionelles Stoppelement, das von einer ersten Art von Rekombinase-Erkennungssequenzen flankiert ist; und
(iii) eine DNA-Sequenz, die nach Transkription den Anti-Sinnstrang eines shRNA-Moleküls ergibt;
(b) ein zweites Segment umfassend in 5' nach 3'- oder 3' nach 5'-Richtung:
(ba) einen Promotor;
(bb) eine zweite DNA-Sequenz umfassend:
(i) eine DNA-Sequenz, die nach Transkription den Sinnstrang eines shRNA-Moleküls ergibt;
(ii) ein transkriptionelles Stoppelement, das von einer zweiten Art von Rekombinase-Erkennungssequenz flankiert ist; und
(iii) eine DNA-Sequenz, die nach Transkription den Anti-Sinnstrang eines shRNA-Moleküls ergibt;
wobei
(i) die erste Art von Rekombinase-Erkennungssequenzen durch eine Rekombinase erkannt und rekombiniert wird, aber nicht mit der zweiten Art von Rekombinase-Erkennungssequenzen rekombiniert wird;
(ii) die zweite Art von Rekombinase-Erkennungssequenzen von der Rekombinase nach (i) erkannt und rekombiniert wird, aber nicht mit den Rekombinase-Erkennungssequenzen der ersten Art rekombiniert wird; und
(iii) die DNA-Sequenz nach (ab) und (bb) unter der Kontrolle der Promotoren nach (aa) und (ba) nach Entfernen der transkriptionellen Stoppelemente nach (ab) und (bb) durch die Aktivität einer Rekombinase exprimiert wird, was zur Transkription des shRNA-Moleküls in einer Zelle führt.

2. Kombination von DNA-Segmenten nach Anspruch 1, wobei die Segmente im gleichen DNA-Molekül enthalten sind.

3. Kombination nach Anspruch 1 oder 2, wobei die Kombination von DNA-Segmenten Teil eines Vektors ist.

4. Kombination von DNA-Segmenten nach einem der Ansprüche 1 bis 3, wobei die Rekombinase-Erkennungssequenzen lox-Sequenzen sind, die eine loxP-Sequenz vom Wildtyp und eine mutierte loxP-Sequenz umfassen.

5. Kombination von DNA-Segmenten nach Anspruch 4, wobei
(a) die Rekombinase eine Cre-Rekombinase ist, die die Sequenz nach SEQ ID NO.: 1 besitzt; oder/und
(b) wobei entweder die erste oder die zweite Art der loxP-Sequenzen die Sequenz nach SEQ ID NO.: 2 besitzt; oder/und
(c) wobei entweder die erste oder die zweite Art der loxP-Sequenzen die Sequenz nach SEQ ID NO.: 3 besitzt.

6. Kombination von DNA-Segmenten nach einem der Ansprüche 2 bis 5, wobei das transkriptionelle Stoppelement Sequenzen enthält, die mit von RNA-Polymerase III angetriebener Transkription interferieren.

7. Kombination von DNA-Segmenten nach einem der Ansprüche 1 bis 6, wobei die Promotoren Promotoren von Genen sind, die durch RNA-Polymerase III transkribiert werden.

8. Kombination von DNA-Segmenten nach einem der Ansprüche 2 bis 7, wobei das DNA-Molekül, das die Kombination von DNA-Segmenten enthält, weitere Elemente umfasst, die die stabile Integration des Moleküls in das Genom eines nicht-menschlichen Tieres erlaubt.

9. Gentechnisch verändertes nicht-menschliches Tier, das transgen hinsichtlich des DNA-Moleküls ist, das die Kombination von DNA-Segmenten nach einem der Ansprüche 2 bis 8 und ein exprimierbares Rekombinasegen enthält.

10. Isolierte eukaryotische Zelle, die mit der Kombination von DNA-Segmenten nach einem der Ansprüche 1 bis 8 gentechnisch verändert ist.

11. In vitro-Verfahren zum gleichzeitigen Knockdown von zwei Genen in einer eukaryotischen Zelle umfassend die Schritte:
(a) Einführen der Kombination von DNA-Segmenten nach einem der Ansprüche 1 bis 8 in eine Zelle;
(b) Ausschneiden der Transkriptionsstoppelemente nach (ab) und (bb) durch die Aktivität einer Rekombinase.

12. In vitro-Verfahren zur Identifikation einer Kombination von zwei Zielgenen als potenzielles Arzneistoffziel umfassend die Schritte:
(a) Bestimmen der unterschiedlichen Expression oder Aktivität von Nukleinsäuremolekülen oder Proteinen in einer Zelle, die Eigenschaften aufweist, die mit einer Krankheit assoziiert sind, und in einer normalen Zelle;
(b) Knockdown von zwei Genen gemäß dem Verfahren nach Anspruch 11 in der Zelle, die Eigenschaften aufweist, die mit einer Krankheit assoziiert sind; und
(c) Bestimmen des Effekts des Knockdown auf die Zelle, die Eigenschaften aufweist, die mit einer Krankheit assoziiert sind; wobei eine Änderung der Krankheitseigenschaften anzeigt, dass die Kombination der zwei Zielgene ein potenzielles Arzneistoffziel darstellt.

13. Pharmazeutische Zusammensetzung umfassend die Kombination von DNA-Segmenten nach einem der Ansprüche 1 bis 8.

14. Kombination von DNA-Segmenten nach einem der Ansprüche 1 bis 8 für die Gentherapie.

## Revendications

1. Combinaison de segments d'ADN comprenant:
(a) un premier segment comprenant dans l'ordre 5' à 3' ou 3' à 5':
(aa) un promoteur;
(ab) une première séquence d'ADN comprenant:
(i) une séquence d'ADN donnant naissance après transcription au brin sens d'une molécule de shRNA;
(ii) un élément d'arrêt de la transcription qui est flanqué d'un premier type de séquences de reconnaissance de recombinase; et
(iii) une séquence d'ADN donnant naissance après la transcription au brin antisens d'une molécule de shRNA;
(b) un second segment comprenant dans l'ordre 5' à 3' ou 3' à 5':
(ba) un promoteur;
(bb) une seconde séquence d'ADN comprenant:
(i) une séquence d'ADN donnant naissance après transcription au brin sens d'une molécule de shRNA;
(ii) un élément d'arrêt de la transcription qui est flanqué d'un second type de séquences de reconnaissance de recombinase; et
(iii) une séquence d'ADN donnant naissance après la transcription au brin antisens d'une molécule de shRNA;
où
(i) ledit premier type de séquences de reconnaissance de recombinase est reconnu et recombiné par une recombinase mais pas recombiné avec ledit second type de séquences de reconnaissance de recombinase;
(ii) ledit second type de séquences de reconnaissance de recombinase est reconnu et recombiné par la recombinase de (i) mais pas recombiné avec ledit premier type de séquences de reconnaissance de recombinase; et
(iii) ladite séquence d'ADN de (ab) et (bb) est exprimée sous le contrôle desdits promoteurs de (aa) et (ba) après élimination desdits éléments d'arrêt de la transcription de (ab) et (bb) par l'activité d'une recombinase, entraînant la transcription de ladite molécule de shRNA dans une cellule.

2. Combinaison de segments d'ADN selon la revendication 1, où lesdits segments sont contenus dans la même molécule d'ADN.

3. Combinaison selon la revendication 1 ou 2, où ladite combinaison de segments d'ADN fait partie d'un vecteur.

4. Combinaison de segments d'ADN selon l'une quelconque des revendications 1 à 3, où lesdites séquences de reconnaissance de recombinase sont des séquences lox englobant une séquence loxP de type sauvage et une séquence loxP mutante.

5. Combinaison de segments d'ADN selon la revendication 4, où
(a) ladite recombinase est une recombinase Cre ayant la séquence de SEQ ID NO: 1; ou/et
(b) où soit le premier soit le second type de séquences loxP a la séquence de SEQ ID NO: 2; ou/et
(c) où soit le premier soit le second type de séquences loxP a la séquence de SEQ ID NO: 3.

6. Combinaison de segments d'ADN selon l'une quelconque des revendications 2 à 5, où l'élément d'arrêt de la transcription contient des séquences qui interfèrent avec la transcription dirigée par l'ARN polymérase III.

7. Combinaison de segments d'ADN selon l'une quelconque des revendications 1 à 6, où les promoteurs sont des promoteurs de gènes transcrits par l'ARN polymérase III.

8. Combinaison de segments d'ADN selon l'une quelconque des revendications 2 à 7, où ladite molécule d'ADN contenant ladite combinaison de segments d'ADN comprend d'autres éléments permettant une intégration stable de ladite molécule dans le génome d'un animal non humain.

9. Animal non-humain génétiquement modifié transgénique pour la molécule d'ADN contenant ladite combinaison de segments d'ADN selon l'une quelconque des revendications 2 à 8 et un gène de recombinase exprimable.

10. Cellule eucaryote isolée génétiquement modifiée avec la combinaison de segments d'ADN selon l'une quelconque des revendications 1 à 8.

11. Procédé *in vitro* d'inactivation simultanée de deux gènes dans une cellule eucaryote comprenant les étapes suivantes:
(a) l'introduction de la combinaison de segments d'ADN selon l'une quelconque des revendications 1 à 8 dans une cellule;
(b) l'excision des éléments d'arrêt de la transcription de (ab) et (bb) par l'intermédiaire de l'activité d'une recombinase.

12. Procédé *in vitro* d'identification d'une combinaison de deux gènes cibles en tant que cible potentielle de médicaments comprenant les étapes suivantes:
(a) la détermination de l'expression ou de l'activité différente de molécules d'acide nucléique ou de protéines dans une cellule présentant des caractéristiques associées à une maladie et dans une cellule normale;
(b) l'inactivation de deux gènes selon le procédé de la revendication 11 dans ladite cellule présentant des caractéristiques associées à une maladie; et
(c) la détermination de l'effet de l'inactivation sur ladite cellule présentant des caractéristiques associées à une maladie; où un changement dans lesdites caractéristiques de la maladie indique que ladite combinaison de deux gènes cibles est une cible potentielle de médicaments.

13. Composition pharmaceutique comprenant la combinaison de segments d'ADN selon l'une quelconque des revendications 1 à 8.

14. Combinaison de segments d'ADN selon l'une quelconque des revendications 1 à 8 pour une thérapie génique.


### SEQUENCE LISTING

<110> Helmholtz Zentrum München - Deutsches Forschungszentrum für Gesundheit und Umwelt (GmbH)
<120> Means and methods for shRNA mediated conditional knockdown of genes
<130> N1905 PCT
<150> US 60/973055
   <151> 2007-09-17
<160> 35
<170> PatentIn version 3.3
<210> 1
   <211> 1029
   <212> DNA
   <213> Enterobacteria phage P1
<400> 1
   atgtccaatt tactgaccgt acaccaaaat ttgcctgcat taccggtcga tgcaacgagt      60
   gatgaggttc gcaagaacct gatggacatg ttcagggatc gccaggcgtt ttctgagcat     120
   acctggaaaa tgcttctgtc cgtttgccgg tcgtgggcgg catggtgcaa gttgaataac     180
   cggaaatggt ttcccgcaga acctgaagat gttcgcgatt atcttctata tcttcaggcg     240
   cgcggtctgg cagtaaaaac tatccagcaa catttgggcc agctaaacat gcttcatcgt     300
   cggtccgggc tgccacgacc aagtgacagc aatgctgttt cactggttat gcggcggatc     360
   cgaaaagaaa acgttgatgc cggtgaacgt gcaaaacagg ctctagcgtt cgaacgcact     420
   gatttcgacc aggttcgttc actcatggaa aatagcgatc gctgccagga tatacgtaat     480
   ctggcatttc tggggattgc ttataacacc ctgttacgta tagccgaaat tgccaggatc     540
   agggttaaag atatctcacg tactgacggt gggagaatgt taatccatat tggcagaacg     600
   aaaacgctgg ttagcaccgc aggtgtagag aaggcactta gcctgggggt aactaaactg     660
   gtcgagcgat ggatttccgt ctctggtgta gctgatgatc cgaataacta cctgttttgc     720
   cgggtcagaa aaaatggtgt tgccgcgcca tctgccacca gccagctatc aactcgcgcc     780
   ctggaaggga tttttgaagc aactcatcga ttgatttacg gcgctaagga tgactctggt     840
   cagagatacc tggcctggtc tggacacagt gcccgtgtcg gagccgcgcg agatatggcc     900
   cgcgctggag tttcaatacc ggagatcatg caagctggtg gctggaccaa tgtaaatatt     960
   gtcatgaact atatccgtaa cctggatagt gaaacagggg caatggtgcg cctgctggaa    1020
   gatggcgat                                                            1029
<210> 2
   <211> 34
   <212> DNA
   <213> Enterobacteria phage P1
<400> 2
   ataacttcgt atagcataca ttatacgaag ttat 34
<210> 3
   <211> 34
   <212> DNA
   <213> artificial sequence
<220>
   <223> lox2272 sequence
<400> 3
   ataacttcgt ataggatacc ttatacgaag ttat 34
<210> 4
   <211> 60
   <212> DNA
   <213> artificial sequence
<220>
   <223> Gsk-3a short hairpin oligo-nucleotide sense
<400> 4
   ctcattcgga gtagtatacc gaagcttggg tatactactc cgaatgagct tttttggaaa 60
<210> 5
   <211> 66
   <212> DNA
   <213> artificial sequence
<220>
   <223> Gsk-3a short hairpin oligo-nucleotide antisense
<400> 5
   cgctcattcg gagtagtata ccgaagcttg ggtatactac tccgaatgag cttttttgga 60
   aagatc 66
<210> 6
   <211> 60
   <212> DNA
   <213> artificial sequence
<220>
   <223> Gsk-3ß short hairpin oligo-nucleotide sense
<400> 6
   ctgtgtgttg gctgaattgt gaagcttgac aattcagcca acacacagct tttttggaaa 60
<210> 7
   <211> 66
   <212> DNA
   <213> artificial sequence
<220>
   <223> Gsk-3ß short hairpin oligo-nucleotide antisense
<400> 7
   cgctgtgtgt tggctgaatt gtgaagcttg acaattcagc caacacacag cttttttgga 60
   aagatc 66
<210> 8
   <211> 3278
   <212> DNA
   <213> artificial sequence
<220>
   <223> pbsu6 vector
<400> 8
   ataaaacctg caggcatgca agcgatcgcg gggccgcccc cttcaccgag ggcctatttc    60
   ccatgattcc ttcatatttg catatacgat acaaggctgt tagagagata attggaatta   120
   atttgactgt aaacacaaag atattagtac aaaatacgtg acgtagaaag taataatttc   180
   ttgggtagtt tgcagtttta aaattatgtt ttaaaatgga ctatcatatg cttaccgtaa   240
   cttgaaagta tttcgatttc ttggctttat atatcttgtg gaaaggacga aacaccggcc   300
   cattcctcct cggatccaag ggtgggcgcg ccaaggcccg cggggccact agttctagag   360
   cggccccaat tcgccctata gtgagtcgta ttacgcgcgc tcactggccg tcgttttaca   420
   acgtcgtgac tgggaaaacc ctggcgttac ccaacttaat cgccttgcag cacatccccc   480
   tttcgccagc tggcgtaata gcgaagaggc ccgcaccgat cgcccttccc aacagttgcg   540
   cagcctgaat ggcgaatggg acgcgccctg tagcggcgca ttaagcgcgg cgggtgtggt   600
   ggttacgcgc agcgtgaccg ctacacttgc cagcgcccta gcgcccgctc ctttcgcttt   660
   cttcccttcc tttctcgcca cgttcgccgg ctttccccgt caagctctaa atcgggggct   720
   ccctttaggg ttccgattta gtgctttacg gcacctcgac cccaaaaaac ttgattaggg   780
   tgatggttca cgtagtgggc catcgccctg atagacggtt tttcgccctt tgacgttgga   840
   gtccacgttc tttaatagtg gactcttgtt ccaaactgga acaacactca accctatctc   900
   ggtctattct tttgatttat aagggatttt gccgatttcg gcctattggt taaaaaatga   960
   gctgatttaa caaaaattta acgcgaattt taacaaaata ttaacgctta caatttaggt  1020
   ggcacttttc ggggaaatgt gcgcggaacc cctatttgtt tatttttcta aatacattca  1080
   aatatgtatc cgctcatgag acaataaccc tgataaatgc ttcaataata ttgaaaaagg  1140
   aagagtatga gtattcaaca tttccgtgtc gcccttattc ccttttttgc ggcattttgc  1200
   cttcctgttt ttgctcaccc agaaacgctg gtgaaagtaa aagatgctga agatcagttg  1260
   ggtgcacgag tgggttacat cgaactggat ctcaacagcg gtaagatcct tgagagtttt  1320
   cgccccgaag aacgttttcc aatgatgagc acttttaaag ttctgctatg tggcgcggta  1380
   ttatcccgta ttgacgccgg gcaagagcaa ctcggtcgcc gcatacacta ttctcagaat  1440
   gacttggttg agtactcacc agtcacagaa aagcatctta cggatggcat gacagtaaga  1500
   gaattatgca gtgctgccat aaccatgagt gataacactg cggccaactt acttctgaca  1560
   acgatcggag gaccgaagga gctaaccgct tttttgcaca acatggggga tcatgtaact  1620
   cgccttgatc gttgggaacc ggagctgaat gaagccatac caaacgacga gcgtgacacc  1680
   acgatgcctg tagcaatggc aacaacgttg cgcaaactat taactggcga actacttact  1740
   ctagcttccc ggcaacaatt aatagactgg atggaggcgg ataaagttgc aggaccactt  1800
   ctgcgctcgg cccttccggc tggctggttt attgctgata aatctggagc cggtgagcgt  1860
   gggtctcgcg gtatcattgc agcactgggg ccagatggta agccctcccg tatcgtagtt 1920
   atctacacga cggggagtca ggcaactatg gatgaacgaa atagacagat cgctgagata 1980
   ggtgcctcac tgattaagca ttggtaactg tcagaccaag tttactcata tatactttag 2040
   attgatttaa aacttcattt ttaatttaaa aggatctagg tgaagatcct ttttgataat 2100
   ctcatgacca aaatccctta acgtgagttt tcgttccact gagcgtcaga ccccgtagaa 2160
   aagatcaaag gatcttcttg agatcctttt tttctgcgcg taatctgctg cttgcaaaca 2220
   aaaaaaccac cgctaccagc ggtggtttgt ttgccggatc aagagctacc aactcttttt 2280
   ccgaaggtaa ctggcttcag cagagcgcag ataccaaata ctgtccttct agtgtagccg 2340
   tagttaggcc accacttcaa gaactctgta gcaccgccta catacctcgc tctgctaatc 2400
   ctgttaccag tggctgctgc cagtggcgat aagtcgtgtc ttaccgggtt ggactcaaga 2460
   cgatagttac cggataaggc gcagcggtcg ggctgaacgg ggggttcgtg cacacagccc 2520
   agcttggagc gaacgaccta caccgaactg agatacctac agcgtgagct atgagaaagc 2580
   gccacgcttc ccgaagggag aaaggcggac aggtatccgg taagcggcag ggtcggaaca 2640
   ggagagcgca cgagggagct tccaggggga aacgcctggt atctttatag tcctgtcggg 2700
   tttcgccacc tctgacttga gcgtcgattt ttgtgatgct cgtcaggggg gcggagccta 2760
   tggaaaaacg ccagcaacgc ggccttttta cggttcctgg ccttttgctg gccttttgct 2820
   cacatgttct ttcctgcgtt atcccctgat tctgtggata accgtattac cgcctttgag 2880
   tgagctgata ccgctcgccg cagccgaacg accgagcgca gcgagtcagt gagcgaggaa 2940
   gcggaagagc gcccaatacg caaaccgcct ctccccgcgc gttggccgat tcattaatgc 3000
   agctggcacg acaggtttcc cgactggaaa gcgggcagtg agcgcaacgc aattaatgtg 3060
   agttagctca ctcattaggc accccaggct ttacacttta tgcttccggc tcgtatgttg 3120
   tgtggaattg tgagcggata acaatttcac acaggaaaca gctatgacca tgattacgcc 3180
   aagcgcgcaa ttaaccctca ctaaagggaa caaaagctgg aaacatgcat gaagttccta 3240
   ttccgaagtt cctattctct agaaagtata ggaacttc 3278
<210> 9
   <211> 883
   <212> DNA
   <213> artificial sequence
<220>
   <223> stop cassette loxP-stop-loxP
<400> 9
   gagtcgactg ataacttcgt atagcataca ttatacgaag ttatggatcc agcttggtag    60
   cgcggtgtat tatacttttt ggaaagaatt cgcccggttc tttttgtcaa gaccgacctg   120
   tccggtgccc tgaatgaact gcaggacgag gcagcgcggc tatcgtggct ggccacgacg   180
   ggcgttcctt gcgcagctgt gctcgacgtt gtcactgaag cgggaaggga ctggctgcta   240
   ttgggcgaag tgccggggca ggatctcctg tcatctcacc ttgctcctgc cgagaaagta   300
   tccatcatgg ctgatgcaat gcggcggctg catacgcttg atccggctac ctgcccattc   360
   gaccaccaag cgaaacatcg catcgagcga gcacgtactc ggatggaagc cggtcttgtc   420
   gatcaggatg atctggacga agagcatcag gggctcgcgc cagccgaact gttcgccagg   480
   ctcaaggcgc gcatgcccga cggcgatgat ctcgtcgtga cccatggcga tgcctgcttg   540
   ccgaatatca tggtggaaaa tggccgcttt tctggattca tcgactgtgg ccggctgggt   600
   gtggcggacc gctatcagga catagcgttg gctacccgtg atattgctga agagcttggc   660
   ggcgaatggg ctgaccgctt cctcgtgctt tacggtatcg ccgctcccga ttcgcagcgc   720
   atcgccttct atcgccttct tgacgagttc ttctgagggg atcaattctc tagcgcctga   780
   tgcggtattt tctccttacg catctgtgcg gtatttcaca ccgcatattt tttggatcca   840
   taacttcgta tagcatacat tatacgaagt tatgactgga ctc                     883
<210> 10
   <211> 883
   <212> DNA
   <213> artificial sequence
<220>
   <223> stop cassette lox2272-stop-lox2272
<400> 10
   gagtcgactg ataacttcgt ataggatacc ttatacgaag ttatggatcc agcttggtag     60
   cgcggtgtat tatacttttt ggaaagaatt cgcccggttc tttttgtcaa gaccgacctg    120
   tccggtgccc tgaatgaact gcaggacgag gcagcgcggc tatcgtggct ggccacgacg    180
   ggcgttcctt gcgcagctgt gctcgacgtt gtcactgaag cgggaaggga ctggctgcta    240
   ttgggcgaag tgccggggca ggatctcctg tcatctcacc ttgctcctgc cgagaaagta    300
   tccatcatgg ctgatgcaat gcggcggctg catacgcttg atccggctac ctgcccattc    360
   gaccaccaag cgaaacatcg catcgagcga gcacgtactc ggatggaagc cggtcttgtc    420
   gatcaggatg atctggacga agagcatcag gggctcgcgc cagccgaact gttcgccagg    480
   ctcaaggcgc gcatgcccga cggcgatgat ctcgtcgtga cccatggcga tgcctgcttg    540
   ccgaatatca tggtggaaaa tggccgcttt tctggattca tcgactgtgg ccggctgggt    600
   gtggcggacc gctatcagga catagcgttg gctacccgtg atattgctga agagcttggc    660
   ggcgaatggg ctgaccgctt cctcgtgctt tacggtatcg ccgctcccga ttcgcagcgc    720
   atcgccttct atcgccttct tgacgagttc ttctgagggg atcaattctc tagcgcctga    780
   tgcggtattt tctccttacg catctgtgcg gtatttcaca ccgcatattt tttggatcca    840
   taacttcgta taggatacct tatacgaagt tatgactgga ctc                      883
<210> 11
   <211> 4190
   <212> DNA
   <213> artificial sequence
<220>
   <223> pbsu6-shGsk-3a-flox2
<400> 11
   ataaaacctg caggcatgca agcgatcgcg gggccgcccc cttcaccgag ggcctatttc 60
   ccatgattcc ttcatatttg catatacgat acaaggctgt tagagagata attggaatta 120
   atttgactgt aaacacaaag atattagtac aaaatacgtg acgtagaaag taataatttc 180
   ttgggtagtt tgcagtttta aaattatgtt ttaaaatgga ctatcatatg cttaccgtaa 240
   cttgaaagta tttcgatttc ttggctttat atatcttgtg gaaaggacga aacaccgctc 300
   attcggagta gtataccgaa gctataactt cgtataggat accttatacg aagttatgga 360
   tccagcttgg tagcgcggtg tattatactt tttggaaaga attcgcccgg ttctttttgt 420
   caagaccgac ctgtccggtg ccctgaatga actgcaggac gaggcagcgc ggctatcgtg 480
   gctggccacg acgggcgttc cttgcgcagc tgtgctcgac gttgtcactg aagcgggaag 540
   ggactggctg ctattgggcg aagtgccggg gcaggatctc ctgtcatctc accttgctcc 600
   tgccgagaaa gtatccatca tggctgatgc aatgcggcgg ctgcatacgc ttgatccggc 660
   tacctgccca ttcgaccacc aagcgaaaca tcgcatcgag cgagcacgta ctcggatgga 720
   agccggtctt gtcgatcagg atgatctgga cgaagagcat caggggctcg cgccagccga 780
   actgttcgcc aggctcaagg cgcgcatgcc cgacggcgat gatctcgtcg tgacccatgg 840
   cgatgcctgc ttgccgaata tcatggtgga aaatggccgc ttttctggat tcatcgactg 900
   tggccggctg ggtgtggcgg accgctatca ggacatagcg ttggctaccc gtgatattgc 960
   tgaagagctt ggcggcgaat gggctgaccg cttcctcgtg ctttacggta tcgccgctcc 1020
   cgattcgcag cgcatcgcct tctatcgcct tcttgacgag ttcttctgag gggatcaatt 1080
   ctctagcgcc tgatgcggta ttttctcctt acgcatctgt gcggtatttc acaccgcata 1140
   ttttttggat ccataacttc gtataggata ccttatacga agttatagct tgggtatact 1200
   actccgaatg agcttttttg gaaagatcca agggtgggcg cgccaaggcc cgcggggcca 1260
   ctagttctag agcggcccca attcgcccta tagtgagtcg tattacgcgc gctcactggc 1320
   cgtcgtttta caacgtcgtg actgggaaaa ccctggcgtt acccaactta atcgccttgc 1380
   agcacatccc cctttcgcca gctggcgtaa tagcgaagag gcccgcaccg atcgcccttc 1440
   ccaacagttg cgcagcctga atggcgaatg ggacgcgccc tgtagcggcg cattaagcgc 1500
   ggcgggtgtg gtggttacgc gcagcgtgac cgctacactt gccagcgccc tagcgcccgc 1560
   tcctttcgct ttcttccctt cctttctcgc cacgttcgcc ggctttcccc gtcaagctct 1620
   aaatcggggg ctccctttag ggttccgatt tagtgcttta cggcacctcg accccaaaaa 1680
   acttgattag ggtgatggtt cacgtagtgg gccatcgccc tgatagacgg tttttcgccc 1740
   tttgacgttg gagtccacgt tctttaatag tggactcttg ttccaaactg gaacaacact 1800
   caaccctatc tcggtctatt cttttgattt ataagggatt ttgccgattt cggcctattg 1860
   gttaaaaaat gagctgattt aacaaaaatt taacgcgaat tttaacaaaa tattaacgct 1920
   tacaatttag gtggcacttt tcggggaaat gtgcgcggaa cccctatttg tttatttttc 1980
   taaatacatt caaatatgta tccgctcatg agacaataac cctgataaat gcttcaataa 2040
   tattgaaaaa ggaagagtat gagtattcaa catttccgtg tcgcccttat tccctttttt 2100
   gcggcatttt gccttcctgt ttttgctcac ccagaaacgc tggtgaaagt aaaagatgct 2160
   gaagatcagt tgggtgcacg agtgggttac atcgaactgg atctcaacag cggtaagatc 2220
   cttgagagtt ttcgccccga agaacgtttt ccaatgatga gcacttttaa agttctgcta 2280
   tgtggcgcgg tattatcccg tattgacgcc gggcaagagc aactcggtcg ccgcatacac 2340
   tattctcaga atgacttggt tgagtactca ccagtcacag aaaagcatct tacggatggc 2400
   atgacagtaa gagaattatg cagtgctgcc ataaccatga gtgataacac tgcggccaac 2460
   ttacttctga caacgatcgg aggaccgaag gagctaaccg cttttttgca caacatgggg 2520
   gatcatgtaa ctcgccttga tcgttgggaa ccggagctga atgaagccat accaaacgac 2580
   gagcgtgaca ccacgatgcc tgtagcaatg gcaacaacgt tgcgcaaact attaactggc 2640
   gaactactta ctctagcttc ccggcaacaa ttaatagact ggatggaggc ggataaagtt 2700
   gcaggaccac ttctgcgctc ggcccttccg gctggctggt ttattgctga taaatctgga 2760
   gccggtgagc gtgggtctcg cggtatcatt gcagcactgg ggccagatgg taagccctcc 2820
   cgtatcgtag ttatctacac gacggggagt caggcaacta tggatgaacg aaatagacag 2880
   atcgctgaga taggtgcctc actgattaag cattggtaac tgtcagacca agtttactca 2940
   tatatacttt agattgattt aaaacttcat ttttaattta aaaggatcta ggtgaagatc 3000
   ctttttgata atctcatgac caaaatccct taacgtgagt tttcgttcca ctgagcgtca 3060
   gaccccgtag aaaagatcaa aggatcttct tgagatcctt tttttctgcg cgtaatctgc 3120
   tgcttgcaaa caaaaaaacc accgctacca gcggtggttt gtttgccgga tcaagagcta 3180
   ccaactcttt ttccgaaggt aactggcttc agcagagcgc agataccaaa tactgtcctt 3240
   ctagtgtagc cgtagttagg ccaccacttc aagaactctg tagcaccgcc tacatacctc 3300
   gctctgctaa tcctgttacc agtggctgct gccagtggcg ataagtcgtg tcttaccggg 3360
   ttggactcaa gacgatagtt accggataag gcgcagcggt cgggctgaac ggggggttcg 3420
   tgcacacagc ccagcttgga gcgaacgacc tacaccgaac tgagatacct acagcgtgag 3480
   ctatgagaaa gcgccacgct tcccgaaggg agaaaggcgg acaggtatcc ggtaagcggc 3540
   agggtcggaa caggagagcg cacgagggag cttccagggg gaaacgcctg gtatctttat 3600
   agtcctgtcg ggtttcgcca cctctgactt gagcgtcgat ttttgtgatg ctcgtcaggg 3660
   gggcggagcc tatggaaaaa cgccagcaac gcggcctttt tacggttcct ggccttttgc 3720
   tggccttttg ctcacatgtt ctttcctgcg ttatcccctg attctgtgga taaccgtatt 3780
   accgcctttg agtgagctga taccgctcgc cgcagccgaa cgaccgagcg cagcgagtca 3840
   gtgagcgagg aagcggaaga gcgcccaata cgcaaaccgc ctctccccgc gcgttggccg 3900
   attcattaat gcagctggca cgacaggttt cccgactgga aagcgggcag tgagcgcaac 3960
   gcaattaatg tgagttagct cactcattag gcaccccagg ctttacactt tatgcttccg 4020
   gctcgtatgt tgtgtggaat tgtgagcgga taacaatttc acacaggaaa cagctatgac 4080
   catgattacg ccaagcgcgc aattaaccct cactaaaggg aacaaaagct ggaaacatgc 4140
   atgaagttcc tattccgaag ttcctattct ctagaaagta taggaacttc 4190
<210> 12
   <211> 4190
   <212> DNA
   <213> artificial sequence
<220>
   <223> pbsu6-shGsk-3ß-flox
<400> 12
   ataaaacctg caggcatgca agcgatcgcg gggccgcccc cttcaccgag ggcctatttc      60
   ccatgattcc ttcatatttg catatacgat acaaggctgt tagagagata attggaatta     120
   atttgactgt aaacacaaag atattagtac aaaatacgtg acgtagaaag taataatttc     180
   ttgggtagtt tgcagtttta aaattatgtt ttaaaatgga ctatcatatg cttaccgtaa     240
   cttgaaagta tttcgatttc ttggctttat atatcttgtg gaaaggacga aacaccgctg     300
   tgtgttggct gaattgtgaa gctataactt cgtatagcat acattatacg aagttatgga     360
   tccagcttgg tagcgcggtg tattatactt tttggaaaga attcgcccgg ttctttttgt     420
   caagaccgac ctgtccggtg ccctgaatga actgcaggac gaggcagcgc ggctatcgtg     480
   gctggccacg acgggcgttc cttgcgcagc tgtgctcgac gttgtcactg aagcgggaag     540
   ggactggctg ctattgggcg aagtgccggg gcaggatctc ctgtcatctc accttgctcc     600
   tgccgagaaa gtatccatca tggctgatgc aatgcggcgg ctgcatacgc ttgatccggc     660
   tacctgccca ttcgaccacc aagcgaaaca tcgcatcgag cgagcacgta ctcggatgga     720
   agccggtctt gtcgatcagg atgatctgga cgaagagcat caggggctcg cgccagccga     780
   actgttcgcc aggctcaagg cgcgcatgcc cgacggcgat gatctcgtcg tgacccatgg     840
   cgatgcctgc ttgccgaata tcatggtgga aaatggccgc ttttctggat tcatcgactg     900
   tggccggctg ggtgtggcgg accgctatca ggacatagcg ttggctaccc gtgatattgc     960
   tgaagagctt ggcggcgaat gggctgaccg cttcctcgtg ctttacggta tcgccgctcc    1020
   cgattcgcag cgcatcgcct tctatcgcct tcttgacgag ttcttctgag gggatcaatt    1080
   ctctagcgcc tgatgcggta ttttctcctt acgcatctgt gcggtatttc acaccgcata    1140
   ttttttggat ccataacttc gtatagcata cattatacga agttatagct tgacaattca    1200
   gccaacacac agcttttttg gaaagatcca agggtgggcg cgccaaggcc cgcggggcca    1260
   ctagttctag agcggcccca attcgcccta tagtgagtcg tattacgcgc gctcactggc    1320
   cgtcgtttta caacgtcgtg actgggaaaa ccctggcgtt acccaactta atcgccttgc    1380
   agcacatccc cctttcgcca gctggcgtaa tagcgaagag gcccgcaccg atcgcccttc    1440
   ccaacagttg cgcagcctga atggcgaatg ggacgcgccc tgtagcggcg cattaagcgc    1500
   ggcgggtgtg gtggttacgc gcagcgtgac cgctacactt gccagcgccc tagcgcccgc    1560
   tcctttcgct ttcttccctt cctttctcgc cacgttcgcc ggctttcccc gtcaagctct 1620
   aaatcggggg ctccctttag ggttccgatt tagtgcttta cggcacctcg accccaaaaa 1680
   acttgattag ggtgatggtt cacgtagtgg gccatcgccc tgatagacgg tttttcgccc 1740
   tttgacgttg gagtccacgt tctttaatag tggactcttg ttccaaactg gaacaacact 1800
   caaccctatc tcggtctatt cttttgattt ataagggatt ttgccgattt cggcctattg 1860
   gttaaaaaat gagctgattt aacaaaaatt taacgcgaat tttaacaaaa tattaacgct 1920
   tacaatttag gtggcacttt tcggggaaat gtgcgcggaa cccctatttg tttatttttc 1980
   taaatacatt caaatatgta tccgctcatg agacaataac cctgataaat gcttcaataa 2040
   tattgaaaaa ggaagagtat gagtattcaa catttccgtg tcgcccttat tccctttttt 2100
   gcggcatttt gccttcctgt ttttgctcac ccagaaacgc tggtgaaagt aaaagatgct 2160
   gaagatcagt tgggtgcacg agtgggttac atcgaactgg atctcaacag cggtaagatc 2220
   cttgagagtt ttcgccccga agaacgtttt ccaatgatga gcacttttaa agttctgcta 2280
   tgtggcgcgg tattatcccg tattgacgcc gggcaagagc aactcggtcg ccgcatacac 2340
   tattctcaga atgacttggt tgagtactca ccagtcacag aaaagcatct tacggatggc 2400
   atgacagtaa gagaattatg cagtgctgcc ataaccatga gtgataacac tgcggccaac 2460
   ttacttctga caacgatcgg aggaccgaag gagctaaccg cttttttgca caacatgggg 2520
   gatcatgtaa ctcgccttga tcgttgggaa ccggagctga atgaagccat accaaacgac 2580
   gagcgtgaca ccacgatgcc tgtagcaatg gcaacaacgt tgcgcaaact attaactggc 2640
   gaactactta ctctagcttc ccggcaacaa ttaatagact ggatggaggc ggataaagtt 2700
   gcaggaccac ttctgcgctc ggcccttccg gctggctggt ttattgctga taaatctgga 2760
   gccggtgagc gtgggtctcg cggtatcatt gcagcactgg ggccagatgg taagccctcc 2820
   cgtatcgtag ttatctacac gacggggagt caggcaacta tggatgaacg aaatagacag 2880
   atcgctgaga taggtgcctc actgattaag cattggtaac tgtcagacca agtttactca 2940
   tatatacttt agattgattt aaaacttcat ttttaattta aaaggatcta ggtgaagatc 3000
   ctttttgata atctcatgac caaaatccct taacgtgagt tttcgttcca ctgagcgtca 3060
   gaccccgtag aaaagatcaa aggatcttct tgagatcctt tttttctgcg cgtaatctgc 3120
   tgcttgcaaa caaaaaaacc accgctacca gcggtggttt gtttgccgga tcaagagcta 3180
   ccaactcttt ttccgaaggt aactggcttc agcagagcgc agataccaaa tactgtcctt 3240
   ctagtgtagc cgtagttagg ccaccacttc aagaactctg tagcaccgcc tacatacctc 3300
   gctctgctaa tcctgttacc agtggctgct gccagtggcg ataagtcgtg tcttaccggg 3360
   ttggactcaa gacgatagtt accggataag gcgcagcggt cgggctgaac ggggggttcg 3420
   tgcacacagc ccagcttgga gcgaacgacc tacaccgaac tgagatacct acagcgtgag 3480
   ctatgagaaa gcgccacgct tcccgaaggg agaaaggcgg acaggtatcc ggtaagcggc 3540
   agggtcggaa caggagagcg cacgagggag cttccagggg gaaacgcctg gtatctttat 3600
   agtcctgtcg ggtttcgcca cctctgactt gagcgtcgat ttttgtgatg ctcgtcaggg 3660
   gggcggagcc tatggaaaaa cgccagcaac gcggcctttt tacggttcct ggccttttgc 3720
   tggccttttg ctcacatgtt ctttcctgcg ttatcccctg attctgtgga taaccgtatt 3780
   accgcctttg agtgagctga taccgctcgc cgcagccgaa cgaccgagcg cagcgagtca 3840
   gtgagcgagg aagcggaaga gcgcccaata cgcaaaccgc ctctccccgc gcgttggccg 3900
   attcattaat gcagctggca cgacaggttt cccgactgga aagcgggcag tgagcgcaac 3960
   gcaattaatg tgagttagct cactcattag gcaccccagg ctttacactt tatgcttccg 4020
   gctcgtatgt tgtgtggaat tgtgagcgga taacaatttc acacaggaaa cagctatgac 4080
   catgattacg ccaagcgcgc aattaaccct cactaaaggg aacaaaagct ggaaacatgc 4140
   atgaagttcc tattccgaag ttcctattct ctagaaagta taggaacttc 4190
<210> 13
   <211> 4380
   <212> DNA
   <213> artificial sequence
<220>
   <223> pRMCE-II
<400> 13
   caattaatgt gagttagctc actcattagg caccccaggc tttacacttt atgcttccgg     60
   ctcgtatgtt gtgtggaatt gtgagcggat aacaatttca cacaggaaac agctatgacc    120
   atgattacgc caagcgcgca attaaccctc actaaaggga acaaaagctg ggtaccgggc    180
   cccccctcga cgcgccgtcg acgtttgatc cccgcggtgc gggtgccagg gcgtgccctt    240
   gggctccccg ggcgcgtact ccacggatca aacgcgctgt tctcctcttc ctcatctccg    300
   ggcctttcga cctgcagcca atatgggatc ggccattgaa caagatggat tgcacgcagg    360
   ttctccggcc gcttgggtgg agaggctatt cggctatgac tgggcacaac agacaatcgg    420
   ctgctctgat gccgccgtgt tccggctgtc agcgcagggg cgcccggttc tttttgtcaa    480
   gaccgacctg tccggtgccc tgaatgaact gcaggacgag gcagcgcggc tatcgtggct    540
   ggccacgacg ggcgttcctt gcgcagctgt gctcgacgtt gtcactgaag cgggaaggga    600
   ctggctgcta ttgggcgaag tgccggggca ggatctcctg tcatctcacc ttgctcctgc    660
   cgagaaagta tccatcatgg ctgatgcaat gcggcggctg catacgcttg atccggctac    720
   ctgcccattc gaccaccaag cgaaacatcg catcgagcga gcacgtactc ggatggaagc    780
   cggtcttgtc gatcaggatg atctggacga agagcatcag gggctcgcgc cagccgaact    840
   gttcgccagg ctcaaggcgc gcatgcccga cggcgatgat ctcgtcgtga cccatggcga    900
   tgcctgcttg ccgaatatca tggtggaaaa tggccgcttt tctggattca tcgactgtgg    960
   ccggctgggt gtggcggacc gctatcagga catagcgttg gctacccgtg atattgctga   1020
   agagcttggc ggcgaatggg ctgaccgctt cctcgtgctt tacggtatcg ccgctcccga   1080
   ttcgcagcgc atcgccttct atcgccttct tgacgagttc ttctgagggg atcaattctc   1140
   tagagctcgc tgatcagcct cgactgtgcc ttctagttgc cagccatctg ttgtttgccc 1200
   ctcccccgtg ccttccttga ccctggaagg tgccactccc actgtccttt cctaataaaa 1260
   tgaggaaatt gcatcgcatt gtctgagtag gtgtcattct attctggggg gtggggtggg 1320
   gcaggacagc aagggggagg attgggaaga caatagcagg catgctgggg atgcggtggg 1380
   ctctatggct tctgaggcgg aaagaaccag ctggggctcg aaaacatgca tgaagttcct 1440
   attccgaagt tcctattctc tagaaagtat aggaacttca taaaacctgc aggcatgcaa 1500
   gcgattaact ttaaataatt ggcattattt aaagttagcg atcgcggccg gcccgcgggg 1560
   cctaacttta aataattggc attatttaaa gttagcgggg ccactagttc tagagcgatc 1620
   cccgcggtgc gggtgccagg gcgtgccctt gggctccccg ggcgcgtact ccacggatcg 1680
   ccaccgcggt ggagctccaa ttcgccctat agtgagtcgt attacgcgcg ctcactggcc 1740
   gtcgttttac aacgtcgtga ctgggaaaac cctggcgtta cccaacttaa tcgccttgca 1800
   gcacatcccc ctttcgccag ctggcgtaat agcgaagagg cccgcaccga tcgcccttcc 1860
   caacagttgc gcagcctgaa tggcgaatgg gacgcgccct gtagcggcgc attaagcgcg 1920
   gcgggtgtgg tggttacgcg cagcgtgacc gctacacttg ccagcgccct agcgcccgct 1980
   cctttcgctt tcttcccttc ctttctcgcc acgttcgccg gctttccccg tcaagctcta 2040
   aatcgggggc tccctttagg gttccgattt agtgctttac ggcacctcga ccccaaaaaa 2100
   cttgattagg gtgatggttc acgtagtggg ccatcgccct gatagacggt ttttcgccct 2160
   ttgacgttgg agtccacgtt ctttaatagt ggactcttgt tccaaactgg aacaacactc 2220
   aaccctatct cggtctattc ttttgattta taagggattt tgccgatttc ggcctattgg 2280
   ttaaaaaatg agctgattta acaaaaattt aacgcgaatt ttaacaaaat attaacgctt 2340
   acaatttagg tggcactttt cggggaaatg tgcgcggaac ccctatttgt ttatttttct 2400
   aaatacattc aaatatgtat ccgctcatga gacaataacc ctgataaatg cttcaataat 2460
   attgaaaaag gaagagtatg agtattcaac atttccgtgt cgcccttatt cccttttttg 2520
   cggcattttg ccttcctgtt tttgctcacc cagaaacgct ggtgaaagta aaagatgctg 2580
   aagatcagtt gggtgcacga gtgggttaca tcgaactgga tctcaacagc ggtaagatcc 2640
   ttgagagttt tcgccccgaa gaacgttttc caatgatgag cacttttaaa gttctgctat 2700
   gtggcgcggt attatcccgt attgacgccg ggcaagagca actcggtcgc cgcatacact 2760
   attctcagaa tgacttggtt gagtactcac cagtcacaga aaagcatctt acggatggca 2820
   tgacagtaag agaattatgc agtgctgcca taaccatgag tgataacact gcggccaact 2880
   tacttctgac aacgatcgga ggaccgaagg agctaaccgc ttttttgcac aacatggggg 2940
   atcatgtaac tcgccttgat cgttgggaac cggagctgaa tgaagccata ccaaacgacg 3000
   agcgtgacac cacgatgcct gtagcaatgg caacaacgtt gcgcaaacta ttaactggcg 3060
   aactacttac tctagcttcc cggcaacaat taatagactg gatggaggcg gataaagttg 3120
   caggaccact tctgcgctcg gcccttccgg ctggctggtt tattgctgat aaatctggag 3180
   ccggtgagcg tgggtctcgc ggtatcattg cagcactggg gccagatggt aagccctccc 3240
   gtatcgtagt tatctacacg acggggagtc aggcaactat ggatgaacga aatagacaga 3300
   tcgctgagat aggtgcctca ctgattaagc attggtaact gtcagaccaa gtttactcat 3360
   atatacttta gattgattta aaacttcatt tttaatttaa aaggatctag gtgaagatcc 3420
   tttttgataa tctcatgacc aaaatccctt aacgtgagtt ttcgttccac tgagcgtcag 3480
   accccgtaga aaagatcaaa ggatcttctt gagatccttt ttttctgcgc gtaatctgct 3540
   gcttgcaaac aaaaaaacca ccgctaccag cggtggtttg tttgccggat caagagctac 3600
   caactctttt tccgaaggta actggcttca gcagagcgca gataccaaat actgtccttc 3660
   tagtgtagcc gtagttaggc caccacttca agaactctgt agcaccgcct acatacctcg 3720
   ctctgctaat cctgttacca gtggctgctg ccagtggcga taagtcgtgt cttaccgggt 3780
   tggactcaag acgatagtta ccggataagg cgcagcggtc gggctgaacg gggggttcgt 3840
   gcacacagcc cagcttggag cgaacgacct acaccgaact gagataccta cagcgtgagc 3900
   tatgagaaag cgccacgctt cccgaaggga gaaaggcgga caggtatccg gtaagcggca 3960
   gggtcggaac aggagagcgc acgagggagc ttccaggggg aaacgcctgg tatctttata 4020
   gtcctgtcgg gtttcgccac ctctgacttg agcgtcgatt tttgtgatgc tcgtcagggg 4080
   ggcggagcct atggaaaaac gccagcaacg cggccttttt acggttcctg gccttttgct 4140
   ggccttttgc tcacatgttc tttcctgcgt tatcccctga ttctgtggat aaccgtatta 4200
   ccgcctttga gtgagctgat accgctcgcc gcagccgaac gaccgagcgc agcgagtcag 4260
   tgagcgagga agcggaagag cgcccaatac gcaaaccgcc tctccccgcg cgttggccga 4320
   ttcattaatg cagctggcac gacaggtttc ccgactggaa agcgggcagt gagcgcaacg 4380
<210> 14
   <211> 5593
   <212> DNA
   <213> artificial sequence
<220>
   <223> pRMCE-II-U6-shGsk-3ß-flox
<400> 14
   attaatgtga gttagctcac tcattaggca ccccaggctt tacactttat gcttccggct      60
   cgtatgttgt gtggaattgt gagcggataa caatttcaca caggaaacag ctatgaccat     120
   gattacgcca agcgcgcaat taaccctcac taaagggaac aaaagctggg taccgggccc     180
   cccctcgacg cgccgtcgac gtttgatccc cgcggtgcgg gtgccagggc gtgcccttgg     240
   gctccccggg cgcgtactcc acggatcaaa cgcgctgttc tcctcttcct catctccggg     300
   cctttcgacc tgcagccaat atgggatcgg ccattgaaca agatggattg cacgcaggtt     360
   ctccggccgc ttgggtggag aggctattcg gctatgactg ggcacaacag acaatcggct     420
   gctctgatgc cgccgtgttc cggctgtcag cgcaggggcg cccggttctt tttgtcaaga     480
   ccgacctgtc cggtgccctg aatgaactgc aggacgaggc agcgcggcta tcgtggctgg     540
   ccacgacggg cgttccttgc gcagctgtgc tcgacgttgt cactgaagcg ggaagggact 600
   ggctgctatt gggcgaagtg ccggggcagg atctcctgtc atctcacctt gctcctgccg 660
   agaaagtatc catcatggct gatgcaatgc ggcggctgca tacgcttgat ccggctacct 720
   gcccattcga ccaccaagcg aaacatcgca tcgagcgagc acgtactcgg atggaagccg 780
   gtcttgtcga tcaggatgat ctggacgaag agcatcaggg gctcgcgcca gccgaactgt 840
   tcgccaggct caaggcgcgc atgcccgacg gcgatgatct cgtcgtgacc catggcgatg 900
   cctgcttgcc gaatatcatg gtggaaaatg gccgcttttc tggattcatc gactgtggcc 960
   ggctgggtgt ggcggaccgc tatcaggaca tagcgttggc tacccgtgat attgctgaag 1020
   agcttggcgg cgaatgggct gaccgcttcc tcgtgcttta cggtatcgcc gctcccgatt 1080
   cgcagcgcat cgccttctat cgccttcttg acgagttctt ctgaggggat caattctcta 1140
   gagctcgctg atcagcctcg actgtgcctt ctagttgcca gccatctgtt gtttgcccct 1200
   cccccgtgcc ttccttgacc ctggaaggtg ccactcccac tgtcctttcc taataaaatg 1260
   aggaaattgc atcgcattgt ctgagtaggt gtcattctat tctggggggt ggggtggggc 1320
   aggacagcaa gggggaggat tgggaagaca atagcaggca tgctggggat gcggtgggct 1380
   ctatggcttc tgaggcggaa agaaccagct ggggctcgaa aacatgcatg aagttcctat 1440
   tccgaagttc ctattctcta gaaagtatag gaacttcata aaacctgcag gcatgcaagc 1500
   gattaacttt aaataattgg cattatttaa agttagcgat cgcggggccg cccccttcac 1560
   cgagggccta tttcccatga ttccttcata tttgcatata cgatacaagg ctgttagaga 1620
   gataattgga attaatttga ctgtaaacac aaagatatta gtacaaaata cgtgacgtag 1680
   aaagtaataa tttcttgggt agtttgcagt tttaaaatta tgttttaaaa tggactatca 1740
   tatgcttacc gtaacttgaa agtatttcga tttcttggct ttatatatct tgtggaaagg 1800
   acgaaacacc gctgtgtgtt ggctgaattg tgaagctata acttcgtata gcatacatta 1860
   tacgaagtta tggatccagc ttggtagcgc ggtgtattat actttttgga aagaattcgc 1920
   ccggttcttt ttgtcaagac cgacctgtcc ggtgccctga atgaactgca ggacgaggca 1980
   gcgcggctat cgtggctggc cacgacgggc gttccttgcg cagctgtgct cgacgttgtc 2040
   actgaagcgg gaagggactg gctgctattg ggcgaagtgc cggggcagga tctcctgtca 2100
   tctcaccttg ctcctgccga gaaagtatcc atcatggctg atgcaatgcg gcggctgcat 2160
   acgcttgatc cggctacctg cccattcgac caccaagcga aacatcgcat cgagcgagca 2220
   cgtactcgga tggaagccgg tcttgtcgat caggatgatc tggacgaaga gcatcagggg 2280
   ctcgcgccag ccgaactgtt cgccaggctc aaggcgcgca tgcccgacgg cgatgatctc 2340
   gtcgtgaccc atggcgatgc ctgcttgccg aatatcatgg tggaaaatgg ccgcttttct 2400
   ggattcatcg actgtggccg gctgggtgtg gcggaccgct atcaggacat agcgttggct 2460
   acccgtgata ttgctgaaga gcttggcggc gaatgggctg accgcttcct cgtgctttac 2520
   ggtatcgccg ctcccgattc gcagcgcatc gccttctatc gccttcttga cgagttcttc 2580
   tgaggggatc aattctctag cgcctgatgc ggtattttct ccttacgcat ctgtgcggta 2640
   tttcacaccg catatttttt ggatccataa cttcgtatag catacattat acgaagttat 2700
   agcttgacaa ttcagccaac acacagcttt tttggaaaga tccaagggtg ggcgcgccaa 2760
   ggcccgcggg gcctaacttt aaataattgg cattatttaa agttagcggg gccactagtt 2820
   ctagagcgat ccccgcggtg cgggtgccag ggcgtgccct tgggctcccc gggcgcgtac 2880
   tccacggatc gccaccgcgg tggagctcca attcgcccta tagtgagtcg tattacgcgc 2940
   gctcactggc cgtcgtttta caacgtcgtg actgggaaaa ccctggcgtt acccaactta 3000
   atcgccttgc agcacatccc cctttcgcca gctggcgtaa tagcgaagag gcccgcaccg 3060
   atcgcccttc ccaacagttg cgcagcctga atggcgaatg ggacgcgccc tgtagcggcg 3120
   cattaagcgc ggcgggtgtg gtggttacgc gcagcgtgac cgctacactt gccagcgccc 3180
   tagcgcccgc tcctttcgct ttcttccctt cctttctcgc cacgttcgcc ggctttcccc 3240
   gtcaagctct aaatcggggg ctccctttag ggttccgatt tagtgcttta cggcacctcg 3300
   accccaaaaa acttgattag ggtgatggtt cacgtagtgg gccatcgccc tgatagacgg 3360
   tttttcgccc tttgacgttg gagtccacgt tctttaatag tggactcttg ttccaaactg 3420
   gaacaacact caaccctatc tcggtctatt cttttgattt ataagggatt ttgccgattt 3480
   cggcctattg gttaaaaaat gagctgattt aacaaaaatt taacgcgaat tttaacaaaa 3540
   tattaacgct tacaatttag gtggcacttt tcggggaaat gtgcgcggaa cccctatttg 3600
   tttatttttc taaatacatt caaatatgta tccgctcatg agacaataac cctgataaat 3660
   gcttcaataa tattgaaaaa ggaagagtat gagtattcaa catttccgtg tcgcccttat 3720
   tccctttttt gcggcatttt gccttcctgt ttttgctcac ccagaaacgc tggtgaaagt 3780
   aaaagatgct gaagatcagt tgggtgcacg agtgggttac atcgaactgg atctcaacag 3840
   cggtaagatc cttgagagtt ttcgccccga agaacgtttt ccaatgatga gcacttttaa 3900
   agttctgcta tgtggcgcgg tattatcccg tattgacgcc gggcaagagc aactcggtcg 3960
   ccgcatacac tattctcaga atgacttggt tgagtactca ccagtcacag aaaagcatct 4020
   tacggatggc atgacagtaa gagaattatg cagtgctgcc ataaccatga gtgataacac 4080
   tgcggccaac ttacttctga caacgatcgg aggaccgaag gagctaaccg cttttttgca 4140
   caacatgggg gatcatgtaa ctcgccttga tcgttgggaa ccggagctga atgaagccat 4200
   accaaacgac gagcgtgaca ccacgatgcc tgtagcaatg gcaacaacgt tgcgcaaact 4260
   attaactggc gaactactta ctctagcttc ccggcaacaa ttaatagact ggatggaggc 4320
   ggataaagtt gcaggaccac ttctgcgctc ggcccttccg gctggctggt ttattgctga 4380
   taaatctgga gccggtgagc gtgggtctcg cggtatcatt gcagcactgg ggccagatgg 4440
   taagccctcc cgtatcgtag ttatctacac gacggggagt caggcaacta tggatgaacg 4500
   aaatagacag atcgctgaga taggtgcctc actgattaag cattggtaac tgtcagacca 4560
   agtttactca tatatacttt agattgattt aaaacttcat ttttaattta aaaggatcta 4620
   ggtgaagatc ctttttgata atctcatgac caaaatccct taacgtgagt tttcgttcca 4680
   ctgagcgtca gaccccgtag aaaagatcaa aggatcttct tgagatcctt tttttctgcg 4740
   cgtaatctgc tgcttgcaaa caaaaaaacc accgctacca gcggtggttt gtttgccgga 4800
   tcaagagcta ccaactcttt ttccgaaggt aactggcttc agcagagcgc agataccaaa 4860
   tactgtcctt ctagtgtagc cgtagttagg ccaccacttc aagaactctg tagcaccgcc 4920
   tacatacctc gctctgctaa tcctgttacc agtggctgct gccagtggcg ataagtcgtg 4980
   tcttaccggg ttggactcaa gacgatagtt accggataag gcgcagcggt cgggctgaac 5040
   ggggggttcg tgcacacagc ccagcttgga gcgaacgacc tacaccgaac tgagatacct 5100
   acagcgtgag ctatgagaaa gcgccacgct tcccgaaggg agaaaggcgg acaggtatcc 5160
   ggtaagcggc agggtcggaa caggagagcg cacgagggag cttccagggg gaaacgcctg 5220
   gtatctttat agtcctgtcg ggtttcgcca cctctgactt gagcgtcgat ttttgtgatg 5280
   ctcgtcaggg gggcggagcc tatggaaaaa cgccagcaac gcggcctttt tacggttcct 5340
   ggccttttgc tggccttttg ctcacatgtt ctttcctgcg ttatcccctg attctgtgga 5400
   taaccgtatt accgcctttg agtgagctga taccgctcgc cgcagccgaa cgaccgagcg 5460
   cagcgagtca gtgagcgagg aagcggaaga gcgcccaata cgcaaaccgc ctctccccgc 5520
   gcgttggccg attcattaat gcagctggca cgacaggttt cccgactgga aagcgggcag 5580
   tgagcgcaac gca 5593
<210> 15
   <211> 6879
   <212> DNA
   <213> artificial sequence
<220>
   <223> pRMCE-II-U6-shGsk-3ß-flox-U6-shGsk-3a-flox2
<400> 15
   caattaatgt gagttagctc actcattagg caccccaggc tttacacttt atgcttccgg 60
   ctcgtatgtt gtgtggaatt gtgagcggat aacaatttca cacaggaaac agctatgacc 120
   atgattacgc caagcgcgca attaaccctc actaaaggga acaaaagctg ggtaccgggc 180
   cccccctcga cgcgccgtcg acgtttgatc cccgcggtgc gggtgccagg gcgtgccctt 240
   gggctccccg ggcgcgtact ccacggatca aacgcgctgt tctcctcttc ctcatctccg 300
   ggcctttcga cctgcagcca atatgggatc ggccattgaa caagatggat tgcacgcagg 360
   ttctccggcc gcttgggtgg agaggctatt cggctatgac tgggcacaac agacaatcgg 420
   ctgctctgat gccgccgtgt tccggctgtc agcgcagggg cgcccggttc tttttgtcaa 480
   gaccgacctg tccggtgccc tgaatgaact gcaggacgag gcagcgcggc tatcgtggct 540
   ggccacgacg ggcgttcctt gcgcagctgt gctcgacgtt gtcactgaag cgggaaggga 600
   ctggctgcta ttgggcgaag tgccggggca ggatctcctg tcatctcacc ttgctcctgc 660
   cgagaaagta tccatcatgg ctgatgcaat gcggcggctg catacgcttg atccggctac 720
   ctgcccattc gaccaccaag cgaaacatcg catcgagcga gcacgtactc ggatggaagc    780
   cggtcttgtc gatcaggatg atctggacga agagcatcag gggctcgcgc cagccgaact    840
   gttcgccagg ctcaaggcgc gcatgcccga cggcgatgat ctcgtcgtga cccatggcga    900
   tgcctgcttg ccgaatatca tggtggaaaa tggccgcttt tctggattca tcgactgtgg    960
   ccggctgggt gtggcggacc gctatcagga catagcgttg gctacccgtg atattgctga   1020
   agagcttggc ggcgaatggg ctgaccgctt cctcgtgctt tacggtatcg ccgctcccga   1080
   ttcgcagcgc atcgccttct atcgccttct tgacgagttc ttctgagggg atcaattctc   1140
   tagagctcgc tgatcagcct cgactgtgcc ttctagttgc cagccatctg ttgtttgccc   1200
   ctcccccgtg ccttccttga ccctggaagg tgccactccc actgtccttt cctaataaaa   1260
   tgaggaaatt gcatcgcatt gtctgagtag gtgtcattct attctggggg gtggggtggg   1320
   gcaggacagc aagggggagg attgggaaga caatagcagg catgctgggg atgcggtggg   1380
   ctctatggct tctgaggcgg aaagaaccag ctggggctcg aaaacatgca tgaagttcct   1440
   attccgaagt tcctattctc tagaaagtat aggaacttca taaaacctgc aggcatgcaa   1500
   gcgattaact ttaaataatt ggcattattt aaagttagcg atcgcggggc cgcccccttc   1560
   accgagggcc tatttcccat gattccttca tatttgcata tacgatacaa ggctgttaga   1620
   gagataattg gaattaattt gactgtaaac acaaagatat tagtacaaaa tacgtgacgt   1680
   agaaagtaat aatttcttgg gtagtttgca gttttaaaat tatgttttaa aatggactat   1740
   catatgctta ccgtaacttg aaagtatttc gatttcttgg ctttatatat cttgtggaaa   1800
   ggacgaaaca ccgctgtgtg ttggctgaat tgtgaagcta taacttcgta tagcatacat   1860
   tatacgaagt tatggatcca gcttggtagc gcggtgtatt atactttttg gaaagaattc   1920
   gcccggttct ttttgtcaag accgacctgt ccggtgccct gaatgaactg caggacgagg   1980
   cagcgcggct atcgtggctg gccacgacgg gcgttccttg cgcagctgtg ctcgacgttg   2040
   tcactgaagc gggaagggac tggctgctat tgggcgaagt gccggggcag gatctcctgt   2100
   catctcacct tgctcctgcc gagaaagtat ccatcatggc tgatgcaatg cggcggctgc   2160
   atacgcttga tccggctacc tgcccattcg accaccaagc gaaacatcgc atcgagcgag   2220
   cacgtactcg gatggaagcc ggtcttgtcg atcaggatga tctggacgaa gagcatcagg   2280
   ggctcgcgcc agccgaactg ttcgccaggc tcaaggcgcg catgcccgac ggcgatgatc   2340
   tcgtcgtgac ccatggcgat gcctgcttgc cgaatatcat ggtggaaaat ggccgctttt   2400
   ctggattcat cgactgtggc cggctgggtg tggcggaccg ctatcaggac atagcgttgg   2460
   ctacccgtga tattgctgaa gagcttggcg gcgaatgggc tgaccgcttc ctcgtgcttt   2520
   acggtatcgc cgctcccgat tcgcagcgca tcgccttcta tcgccttctt gacgagttct   2580
   tctgagggga tcaattctct agcgcctgat gcggtatttt ctccttacgc atctgtgcgg   2640
   tatttcacac cgcatatttt ttggatccat aacttcgtat agcatacatt atacgaagtt   2700
   atagcttgac aattcagcca acacacagct tttttggaaa gatccaaggg tgggcgcgcc   2760
   aaggcccgcg gggcctaact ttaaataatt ggcattattt aaagttagcg gggccactag 2820
   aaagtatagg aacttcataa aacctgcagg catgcaagcg atcgcggggc cgcccccttc 2880
   accgagggcc tatttcccat gattccttca tatttgcata tacgatacaa ggctgttaga 2940
   gagataattg gaattaattt gactgtaaac acaaagatat tagtacaaaa tacgtgacgt 3000
   agaaagtaat aatttcttgg gtagtttgca gttttaaaat tatgttttaa aatggactat 3060
   catatgctta ccgtaacttg aaagtatttc gatttcttgg ctttatatat cttgtggaaa 3120
   ggacgaaaca ccgctcattc ggagtagtat accgaagcta taacttcgta taggatacct 3180
   tatacgaagt tatggatcca gcttggtagc gcggtgtatt atactttttg gaaagaattc 3240
   gcccggttct ttttgtcaag accgacctgt ccggtgccct gaatgaactg caggacgagg 3300
   cagcgcggct atcgtggctg gccacgacgg gcgttccttg cgcagctgtg ctcgacgttg 3360
   tcactgaagc gggaagggac tggctgctat tgggcgaagt gccggggcag gatctcctgt 3420
   catctcacct tgctcctgcc gagaaagtat ccatcatggc tgatgcaatg cggcggctgc 3480
   atacgcttga tccggctacc tgcccattcg accaccaagc gaaacatcgc atcgagcgag 3540
   cacgtactcg gatggaagcc ggtcttgtcg atcaggatga tctggacgaa gagcatcagg 3600
   ggctcgcgcc agccgaactg ttcgccaggc tcaaggcgcg catgcccgac ggcgatgatc 3660
   tcgtcgtgac ccatggcgat gcctgcttgc cgaatatcat ggtggaaaat ggccgctttt 3720
   ctggattcat cgactgtggc cggctgggtg tggcggaccg ctatcaggac atagcgttgg 3780
   ctacccgtga tattgctgaa gagcttggcg gcgaatgggc tgaccgcttc ctcgtgcttt 3840
   acggtatcgc cgctcccgat tcgcagcgca tcgccttcta tcgccttctt gacgagttct 3900
   tctgagggga tcaattctct agcgcctgat gcggtatttt ctccttacgc atctgtgcgg 3960
   tatttcacac cgcatatttt ttggatccat aacttcgtat aggatacctt atacgaagtt 4020
   atagcttggg tatactactc cgaatgagct tttttggaaa gatccaaggg tgggcgcgcc 4080
   aaggcccgcg gggccactag ttctagttct agagcgatcc ccgcggtgcg ggtgccaggg 4140
   cgtgcccttg ggctccccgg gcgcgtactc cacggatcgc caccgcggtg gagctccaat 4200
   tcgccctata gtgagtcgta ttacgcgcgc tcactggccg tcgttttaca acgtcgtgac 4260
   tgggaaaacc ctggcgttac ccaacttaat cgccttgcag cacatccccc tttcgccagc 4320
   tggcgtaata gcgaagaggc ccgcaccgat cgcccttccc aacagttgcg cagcctgaat 4380
   ggcgaatggg acgcgccctg tagcggcgca ttaagcgcgg cgggtgtggt ggttacgcgc 4440
   agcgtgaccg ctacacttgc cagcgcccta gcgcccgctc ctttcgcttt cttcccttcc 4500
   tttctcgcca cgttcgccgg ctttccccgt caagctctaa atcgggggct ccctttaggg 4560
   ttccgattta gtgctttacg gcacctcgac cccaaaaaac ttgattaggg tgatggttca 4620
   cgtagtgggc catcgccctg atagacggtt tttcgccctt tgacgttgga gtccacgttc 4680
   tttaatagtg gactcttgtt ccaaactgga acaacactca accctatctc ggtctattct 4740
   tttgatttat aagggatttt gccgatttcg gcctattggt taaaaatgaa gctgatttaa 4800
   caaaaattta acgcgaattt taacaaaata ttaacgctta caatttaggt ggcacttttc 4860
   ggggaaatgt gcgcggaacc cctatttgtt tatttttcta aatacattca aatatgtatc 4920
   cgctcatgag acaataaccc tgataaatgc ttcaataata ttgaaaaagg aagagtatga 4980
   gtattcaaca tttccgtgtc gcccttattc ccttttttgc ggcattttgc cttcctgttt 5040
   ttgctcaccc agaaacgctg gtgaaagtaa aagatgctga agatcagttg ggtgcacgag 5100
   tgggttacat cgaactggat ctcaacagcg gtaagatcct tgagagtttt cgccccgaag 5160
   aacgttttcc aatgatgagc acttttaaag ttctgctatg tggcgcggta ttatcccgta 5220
   ttgacgccgg gcaagagcaa ctcggtcgcc gcatacacta ttctcagaat gacttggttg 5280
   agtactcacc agtcacagaa aagcatctta cggatggcat gacagtaaga gaattatgca 5340
   gtgctgccat aaccatgagt gataacactg cggccaactt acttctgaca acgatcggag 5400
   gaccgaagga gctaaccgct tttttgcaca acatggggga tcatgtaact cgccttgatc 5460
   gttgggaacc ggagctgaat gaagccatac caaacgacga gcgtgacacc acgatgcctg 5520
   tagcaatggc aacaacgttg cgcaaactat taactggcga actacttact ctagcttccc 5580
   ggcaacaatt aatagactgg atggaggcgg ataaagttgc aggaccactt ctgcgctcgg 5640
   cccttccggc tggctggttt attgctgata aatctggagc cggtgagcgt gggtctcgcg 5700
   gtatcattgc agcactgggg ccagatggta agccctcccg tatcgtagtt atctacacga 5760
   cggggagtca ggcaactatg gatgaacgaa atagacagat cgctgagata ggtgcctcac 5820
   tgattaagca ttggtaactg tcagaccaag tttactcata tatactttag attgatttaa 5880
   aacttcattt ttaatttaaa aggatctagg tgaagatcct ttttgataat ctcatgacca 5940
   aaatccctta acgtgagttt tcgttccact gagcgtcaga ccccgtagaa aagatcaaag 6000
   gatcttcttg agatcctttt tttctgcgcg taatctgctg cttgcaaaca aaaaaaccac 6060
   cgctaccagc ggtggtttgt ttgccggatc aagagctacc aactcttttt ccgaaggtaa 6120
   ctggcttcag cagagcgcag ataccaaata ctgtccttct agtgtagccg tagttaggcc 6180
   accacttcaa gaactctgta gcaccgccta catacctcgc tctgctaatc ctgttaccag 6240
   tggctgctgc cagtggcgat aagtcgtgtc ttaccgggtt ggactcaaga cgatagttac 6300
   cggataaggc gcagcggtcg ggctgaacgg ggggttcgtg cacacagccc agcttggagc 6360
   gaacgaccta caccgaactg agatacctac agcgtgagct atgagaaagc gccacgcttc 6420
   ccgaagggag aaaggcggac aggtatccgg taagcggcag ggtcggaaca ggagagcgca 6480
   cgagggagct tccaggggga aacgcctggt atctttatag tcctgtcggg tttcgccacc 6540
   tctgacttga gcgtcgattt ttgtgatgct cgtcaggggg gcggagccta tggaaaaacg 6600
   ccagcaacgc ggccttttta cggttcctgg ccttttgctg gccttttgct cacatgttct 6660
   ttcctgcgtt atcccctgat tctgtggata accgtattac cgcctttgag tgagctgata 6720
   ccgctcgccg cagccgaacg accgagcgca gcgagtcagt gagcgaggaa gcggaagagc 6780
   gcccaatacg caaaccgcct ctccccgcgc gttggccgat tcattaatgc agctggcacg 6840
   acaggtttcc cgactggaaa gcgggcagtg agcgcaacg 6879
<210> 16
   <211> 6576
   <212> DNA
   <213> artificial sequence
<220>
   <223> pCAG-C31Int-bpA
<400> 16
   tcgcgcgttt cggtgatgac ggtgaaaacc tctgacacat gcagctcccg gagacggtca 60
   cagcttgtct gtaagcggat gccgggagca gacaagcccg tcagggcgcg tcagcgggtg 120
   ttggcgggtg tcggggctgg cttaactatg cggcatcaga gcagattgta ctgagagtgc 180
   accatatgcg gtgtgaaata ccgcacagat gcgtaaggag aaaataccgc atcaggcgcc 240
   attcgccatt caggctgcgc aactgttggg aagggcgatc ggtgcgggcc tcttcgctat 300
   tacgccagct ggcgaaaggg ggatgtgctg caaggcgatt aagttgggta acgccagggt 360
   tttcccagtc acgacgttgt aaaacgacgg ccagtgaatt cgagctcggt acccgggggc 420
   gcgccggatc tcgacattga ttattgacta gttattaata gtaatcaatt acggggtcat 480
   tagttcatag cccatatatg gagttccgcg ttacataact tacggtaaat ggcccgcctg 540
   gctgaccgcc caacgacccc cgcccattga cgtcaataat gacgtatgtt cccatagtaa 600
   cgccaatagg gactttccat tgacgtcaat gggtggacta tttacggtaa actgcccact 660
   tggcagtaca tcaagtgtat catatgccaa gtacgccccc tattgacgtc aatgacggta 720
   aatggcccgc ctggcattat gcccagtaca tgaccttatg ggactttcct acttggcagt 780
   acatctacgt attagtcatc gctattacca tgggtcgagg tgagccccac gttctgcttc 840
   actctcccca tctccccccc ctccccaccc ccaattttgt atttatttat tttttaatta 900
   ttttgtgcag cgatgggggc gggggggggg ggggcgcgcg ccaggcgggg cggggcgggg 960
   cgaggggcgg ggcggggcga ggcggagagg tgcggcggca gccaatcaga gcggcgcgct 1020
   ccgaaagttt ccttttatgg cgaggcggcg gcggcggcgg ccctataaaa agcgaagcgc 1080
   gcggcgggcg ggagtcgctg cgttgccttc gccccgtgcc ccgctccgcg ccgcctcgcg 1140
   ccgcccgccc cggctctgac tgaccgcgtt actcccacag gtgagcgggc gggacggccc 1200
   ttctcctccg ggctgtaatt agcgcttggt ttaatgacgg ctcgtttctt ttctgtggct 1260
   gcgtgaaagc cttaaagggc tccgggaggg ccctttgtgc gggggggagc ggctcggggg 1320
   gtgcgtgcgt gtgtgtgtgc gtggggagcg ccgcgtgcgg cccgcgctgc ccggcggctg 1380
   tgagcgctgc gggcgcggcg cggggctttg tgcgctccgc gtgtgcgcga ggggagcgcg 1440
   gccgggggcg gtgccccgcg gtgcgggggg gctgcgaggg gaacaaaggc tgcgtgcggg 1500
   gtgtgtgcgt gggggggtga gcagggggtg tgggcgcggc ggtcgggctg taaccccccc 1560
   ctgcaccccc ctccccgagt tgctgagcac ggcccggctt cgggtgcggg gctccgtgcg 1620
   gggcgtggcg cggggctcgc cgtgccgggc ggggggtggc ggcaggtggg ggtgccgggc 1680
   ggggcggggc cgcctcgggc cggggagggc tcgggggagg ggcgcggcgg ccccggagcg 1740
   ccggcggctg tcgaggcgcg gcgagccgca gccattgcct tttatggtaa tcgtgcgaga 1800
   gggcgcaggg acttcctttg tcccaaatct ggcggagccg aaatctggga ggcgccgccg 1860
   caccccctct agcgggcgcg ggcgaagcgg tgcggcgccg gcaggaagga aatgggcggg 1920
   gagggccttc gtgcgtcgcc gcgccgccgt ccccttctcc atctccagcc tcggggctgc 1980
   cgcaggggga cggctgcctt cgggggggac ggggcagggc ggggttcggc ttctggcgtg 2040
   tgaccggcgg ctctagagcc tctgctaacc atgttcatgc cttcttcttt ttcctacaga 2100
   tccttaatta agtctagacc gatatgacac aaggggttgt gaccggggtg gacacgtacg 2160
   cgggtgctta cgaccgtcag tcgcgcgagc gcgagaattc gagcgcagca agcccagcga 2220
   cacagcgtag cgccaacgaa gacaaggcgg ccgaccttca gcgcgaagtc gagcgcgacg 2280
   ggggccggtt caggttcgtc gggcatttca gcgaagcgcc gggcacgtcg gcgttcggga 2340
   cggcggagcg cccggagttc gaacgcatcc tgaacgaatg ccgcgccggg cggctcaaca 2400
   tgatcattgt ctatgacgtg tcgcgcttct cgcgcctgaa ggtcatggac gcgattccga 2460
   ttgtctcgga attgctcgcc ctgggcgtga cgattgtttc cactcaggaa ggcgtcttcc 2520
   ggcagggaaa cgtcatggac ctgattcacc tgattatgcg gctcgacgcg tcgcacaaag 2580
   aatcttcgct gaagtcggcg aagattctcg acacgaagaa ccttcagcgc gaattgggcg 2640
   ggtacgtcgg cgggaaggcg ccttacggct tcgagcttgt ttcggagacg aaggagatca 2700
   cgcgcaacgg ccgaatggtc aatgtcgtca tcaacaagct tgcgcactcg accactcccc 2760
   ttaccggacc cttcgagttc gagcccgacg taatccggtg gtggtggcgt gagatcaaga 2820
   cgcacaaaca ccttcccttc aagccgggca gtcaagccgc cattcacccg ggcagcatca 2880
   cggggctttg taagcgcatg gacgctgacg ccgtgccgac ccggggcgag acgattggga 2940
   agaagaccgc ttcaagcgcc tgggacccgg caaccgttat gcgaatcctt cgggacccgc 3000
   gtattgcggg cttcgccgct gaggtgatct acaagaagaa gccggacggc acgccgacca 3060
   cgaagattga gggttaccgc attcagcgcg acccgatcac gctccggccg gtcgagcttg 3120
   attgcggacc gatcatcgag cccgctgagt ggtatgagct tcaggcgtgg ttggacggca 3180
   gggggcgcgg caaggggctt tcccgggggc aagccattct gtccgccatg gacaagctgt 3240
   actgcgagtg tggcgccgtc atgacttcga agcgcgggga agaatcgatc aaggactctt 3300
   accgctgccg tcgccggaag gtggtcgacc cgtccgcacc tgggcagcac gaaggcacgt 3360
   gcaacgtcag catggcggca ctcgacaagt tcgttgcgga acgcatcttc aacaagatca 3420
   ggcacgccga aggcgacgaa gagacgttgg cgcttctgtg ggaagccgcc cgacgcttcg 3480
   gcaagctcac tgaggcgcct gagaagagcg gcgaacgggc gaaccttgtt gcggagcgcg 3540
   ccgacgccct gaacgccctt gaagagctgt acgaagaccg cgcggcaggc gcgtacgacg 3600
   gacccgttgg caggaagcac ttccggaagc aacaggcagc gctgacgctc cggcagcaag 3660
   gggcggaaga gcggcttgcc gaacttgaag ccgccgaagc cccgaagctt ccccttgacc 3720
   aatggttccc cgaagacgcc gacgctgacc cgaccggccc taagtcgtgg tgggggcgcg 3780
   cgtcagtaga cgacaagcgc gtgttcgtcg ggctcttcgt agacaagatc gttgtcacga 3840
   agtcgactac gggcaggggg cagggaacgc ccatcgagaa gcgcgcttcg atcacgtggg 3900
   cgaagccgcc gaccgacgac gacgaagacg acgcccagga cggcacggaa gacgtagcgg 3960
   cgcctaagaa gaagaggaag gtttagtcta gagtcgactg tttctagagc tcgctgatca 4020
   gcctcgactg tgccttctag ttgccagcca tctgttgttt gcccctcccc cgtgccttcc 4080
   ttgaccctgg aaggtgccac tcccactgtc ctttcctaat aaaatgagga aattgcatcg 4140
   cattgtctga gtaggtgtca ttctattctg gggggtgggg tggggcagga cagcaagggg 4200
   gaggattggg aagacaatag caggcatgct ggggatgcgg tgggctctat ggcttctgag 4260
   gcggaaagaa ccagctgggg ctcgagatcc actagttcta gcctcgaggc tagagcggcc 4320
   aaacctgcag gcatgcaagc ttggcgtaat catggtcata gctgtttcct gtgtgaaatt 4380
   gttatccgct cacaattcca cacaacatac gagccggaag cataaagtgt aaagcctggg 4440
   gtgcctaatg agtgagctaa ctcacattaa ttgcgttgcg ctcactgccc gctttccagt 4500
   cgggaaacct gtcgtgccag ctgcattaat gaatcggcca acgcgcgggg agaggcggtt 4560
   tgcgtattgg gcgctcttcc gcttcctcgc tcactgactc gctgcgctcg gtcgttcggc 4620
   tgcggcgagc ggtatcagct cactcaaagg cggtaatacg gttatccaca gaatcagggg 4680
   ataacgcagg aaagaacatg tgagcaaaag gccagcaaaa ggccaggaac cgtaaaaagg 4740
   ccgcgttgct ggcgtttttc cataggctcc gcccccctga cgagcatcac aaaaatcgac 4800
   gctcaagtca gaggtggcga aacccgacag gactataaag ataccaggcg tttccccctg 4860
   gaagctccct cgtgcgctct cctgttccga ccctgccgct taccggatac ctgtccgcct 4920
   ttctcccttc gggaagcgtg gcgctttctc atagctcacg ctgtaggtat ctcagttcgg 4980
   tgtaggtcgt tcgctccaag ctgggctgtg tgcacgaacc ccccgttcag cccgaccgct 5040
   gcgccttatc cggtaactat cgtcttgagt ccaacccggt aagacacgac ttatcgccac 5100
   tggcagcagc cactggtaac aggattagca gagcgaggta tgtaggcggt gctacagagt 5160
   tcttgaagtg gtggcctaac tacggctaca ctagaaggac agtatttggt atctgcgctc 5220
   tgctgaagcc agttaccttc ggaaaaagag ttggtagctc ttgatccggc aaacaaacca 5280
   ccgctggtag cggtggtttt tttgtttgca agcagcagat tacgcgcaga aaaaaaggat 5340
   ctcaagaaga tcctttgatc ttttctacgg ggtctgacgc tcagtggaac gaaaactcac 5400
   gttaagggat tttggtcatg agattatcaa aaaggatctt cacctagatc cttttaaatt 5460
   aaaaatgaag ttttaaatca atctaaagta tatatgagta aacttggtct gacagttacc 5520
   aatgcttaat cagtgaggca cctatctcag cgatctgtct atttcgttca tccatagttg 5580
   cctgactccc cgtcgtgtag ataactacga tacgggaggg cttaccatct ggccccagtg 5640
   ctgcaatgat accgcgagac ccacgctcac cggctccaga tttatcagca ataaaccagc 5700
   cagccggaag ggccgagcgc agaagtggtc ctgcaacttt atccgcctcc atccagtcta 5760
   ttaattgttg ccgggaagct agagtaagta gttcgccagt taatagtttg cgcaacgttg 5820
   ttgccattgc tacaggcatc gtggtgtcac gctcgtcgtt tggtatggct tcattcagct 5880
   ccggttccca acgatcaagg cgagttacat gatcccccat gttgtgcaaa aaagcggtta 5940
   gctccttcgg tcctccgatc gttgtcagaa gtaagttggc cgcagtgtta tcactcatgg 6000
   ttatggcagc actgcataat tctcttactg tcatgccatc cgtaagatgc ttttctgtga 6060
   ctggtgagta ctcaaccaag tcattctgag aatagtgtat gcggcgaccg agttgctctt 6120
   gcccggcgtc aatacgggat aataccgcgc cacatagcag aactttaaaa gtgctcatca 6180
   ttggaaaacg ttcttcgggg cgaaaactct caaggatctt accgctgttg agatccagtt 6240
   cgatgtaacc cactcgtgca cccaactgat cttcagcatc ttttactttc accagcgttt 6300
   ctgggtgagc aaaaacagga aggcaaaatg ccgcaaaaaa gggaataagg gcgacacgga 6360
   aatgttgaat actcatactc ttcctttttc aatattattg aagcatttat cagggttatt 6420
   gtctcatgag cggatacata tttgaatgta tttagaaaaa taaacaaata ggggttccgc 6480
   gcacatttcc ccgaaaagtg ccacctgacg tctaagaaac cattattatc atgacattaa 6540
   cctataaaaa taggcgtatc acgaggccct ttcgtc 6576
<210> 17
   <211> 6081
   <212> DNA
   <213> artificial sequence
<220>
   <223> pCAG-Cre-bpA
<400> 17
   gggtaccggg ccccccctcg aggtcgacgg tatcgataag cttgatatcg aattcgagct 60
   cggtacccgg gggcgcgccg gatctcgaca ttgattattg actagttatt aatagtaatc 120
   aattacgggg tcattagttc atagcccata tatggagttc cgcgttacat aacttacggt 180
   aaatggcccg cctggctgac cgcccaacga cccccgccca ttgacgtcaa taatgacgta 240
   tgttcccata gtaacgccaa tagggacttt ccattgacgt caatgggtgg actatttacg 300
   gtaaactgcc cacttggcag tacatcaagt gtatcatatg ccaagtacgc cccctattga 360
   cgtcaatgac ggtaaatggc ccgcctggca ttatgcccag tacatgacct tatgggactt 420
   tcctacttgg cagtacatct acgtattagt catcgctatt accatgggtc gaggtgagcc 480
   ccacgttctg cttcactctc cccatctccc ccccctcccc acccccaatt ttgtatttat 540
   ttatttttta attattttgt gcagcgatgg gggcgggggg ggggggggcg cgcgccaggc 600
   ggggcggggc ggggcgaggg gcggggcggg gcgaggcgga gaggtgcggc ggcagccaat 660
   cagagcggcg cgctccgaaa gtttcctttt atggcgaggc ggcggcggcg gcggccctat 720
   aaaaagcgaa gcgcgcggcg ggcgggagtc gctgcgttgc cttcgccccg tgccccgctc 780
   cgcgccgcct cgcgccgccc gccccggctc tgactgaccg cgttactccc acaggtgagc 840
   gggcgggacg gcccttctcc tccgggctgt aattagcgct tggtttaatg acggctcgtt 900
   tcttttctgt ggctgcgtga aagccttaaa gggctccggg agggcccttt gtgcgggggg 960
   gagcggctcg gggggtgcgt gcgtgtgtgt gtgcgtgggg agcgccgcgt gcggcccgcg 1020
   ctgcccggcg gctgtgagcg ctgcgggcgc ggcgcggggc tttgtgcgct ccgcgtgtgc 1080
   gcgaggggag cgcggccggg ggcggtgccc cgcggtgcgg gggggctgcg aggggaacaa 1140
   aggctgcgtg cggggtgtgt gcgtgggggg gtgagcaggg ggtgtgggcg cggcggtcgg 1200
   gctgtaaccc ccccctgcac ccccctcccc gagttgctga gcacggcccg gcttcgggtg 1260
   cggggctccg tgcggggcgt ggcgcggggc tcgccgtgcc gggcgggggg tggcggcagg 1320
   tgggggtgcc gggcggggcg gggccgcctc gggccgggga gggctcgggg gaggggcgcg 1380
   gcggccccgg agcgccggcg gctgtcgagg cgcggcgagc cgcagccatt gccttttatg 1440
   gtaatcgtgc gagagggcgc agggacttcc tttgtcccaa atctggcgga gccgaaatct 1500
   gggaggcgcc gccgcacccc ctctagcggg cgcgggcgaa gcggtgcggc gccggcagga 1560
   aggaaatggg cggggagggc cttcgtgcgt cgccgcgccg ccgtcccctt ctccatctcc 1620
   agcctcgggg ctgccgcagg gggacggctg ccttcggggg ggacggggca gggcggggtt 1680
   cggcttctgg cgtgtgaccg gcggctctag agcctctgct aaccatgttc atgccttctt 1740
   ctttttccta cagatcctta attaagtcta gagtcgactg tttaaacctg cagctcgagg 1800
   tcgaccatgc ccaagaagaa gaggaaggtg tccaatttac tgaccgtaca ccaaaatttg 1860
   cctgcattac cggtcgatgc aacgagtgat gaggttcgca agaacctgat ggacatgttc 1920
   agggatcgcc aggcgttttc tgagcatacc tggaaaatgc ttctgtccgt ttgccggtcg 1980
   tgggcggcat ggtgcaagtt gaataaccgg aaatggtttc ccgcagaacc tgaagatgtt 2040
   cgcgattatc ttctatatct tcaggcgcgc ggtctggcag taaaaactat ccagcaacat 2100
   ttgggccagc taaacatgct tcatcgtcgg tccgggctgc cacgaccaag tgacagcaat 2160
   gctgtttcac tggttatgcg gcggatccga aaagaaaacg ttgatgccgg tgaacgtgca 2220
   aaacaggctc tagcgttcga acgcactgat ttcgaccagg ttcgttcact catggaaaat 2280
   agcgatcgct gccaggatat acgtaatctg gcatttctgg ggattgctta taacaccctg 2340
   ttacgtatag ccgaaattgc caggatcagg gttaaagata tctcacgtac tgacggtggg 2400
   agaatgttaa tccatattgg cagaacgaaa acgctggtta gcaccgcagg tgtagagaag 2460
   gcacttagcc tgggggtaac taaactggtc gagcgatgga tttccgtctc tggtgtagct 2520
   gatgatccga ataactacct gttttgccgg gtcagaaaaa atggtgttgc cgcgccatct 2580
   gccaccagcc agctatcaac tcgcgccctg gaagggattt ttgaagcaac tcatcgattg 2640
   atttacggcg ctaaggatga ctctggtcag agatacctgg cctggtctgg acacagtgcc 2700
   cgtgtcggag ccgcgcgaga tatggcccgc gctggagttt caataccgga gatcatgcaa 2760
   gctggtggct ggaccaatgt aaatattgtc atgaactata tccgtaacct ggatagtgaa 2820
   acaggggcaa tggtgcgcct gctggaagat ggcgattagc cattaacgcg taaatgattg 2880
   cagatccact agttctagag ctcgctgatc agcctcgact gtgccttcta gttgccagcc 2940
   atctgttgtt tgcccctccc ccgtgccttc cttgaccctg gaaggtgcca ctcccactgt 3000
   cctttcctaa taaaatgagg aaattgcatc gcattgtctg agtaggtgtc attctattct 3060
   ggggggtggg gtggggcagg acagcaaggg ggaggattgg gaagacaata gcaggcatgc 3120
   tggggatgcg gtgggctcta tggcttctga ggcggaaaga accagctggg gctcgagatc 3180
   cactagttct agcctcgagg ctagagcggc cgccaccgcg gtggagctcc aattcgccct 3240
   atagtgagtc gtattacgcg cgctcactgg ccgtcgtttt acaacgtcgt gactgggaaa 3300
   accctggcgt tacccaactt aatcgccttg cagcacatcc ccctttcgcc agctggcgta 3360
   atagcgaaga ggcccgcacc gatcgccctt cccaacagtt gcgcagcctg aatggcgaat 3420
   gggacgcgcc ctgtagcggc gcattaagcg cggcgggtgt ggtggttacg cgcagcgtga 3480
   ccgctacact tgccagcgcc ctagcgcccg ctcctttcgc tttcttccct tcctttctcg 3540
   ccacgttcgc cggctttccc cgtcaagctc taaatcgggg gctcccttta gggttccgat 3600
   ttagtgcttt acggcacctc gaccccaaaa aacttgatta gggtgatggt tcacgtagtg 3660
   ggccatcgcc ctgatagacg gtttttcgcc ctttgacgtt ggagtccacg ttctttaata 3720
   gtggactctt gttccaaact ggaacaacac tcaaccctat ctcggtctat tcttttgatt 3780
   tataagggat tttgccgatt tcggcctatt ggttaaaaaa tgagctgatt taacaaaaat 3840
   ttaacgcgaa ttttaacaaa atattaacgc ttacaattta ggtggcactt ttcggggaaa 3900
   tgtgcgcgga acccctattt gtttattttt ctaaatacat tcaaatatgt atccgctcat 3960
   gagacaataa ccctgataaa tgcttcaata atattgaaaa aggaagagta tgagtattca 4020
   acatttccgt gtcgccctta ttcccttttt tgcggcattt tgccttcctg tttttgctca 4080
   cccagaaacg ctggtgaaag taaaagatgc tgaagatcag ttgggtgcac gagtgggtta 4140
   catcgaactg gatctcaaca gcggtaagat ccttgagagt tttcgccccg aagaacgttt 4200
   tccaatgatg agcactttta aagttctgct atgtggcgcg gtattatccc gtattgacgc 4260
   cgggcaagag caactcggtc gccgcataca ctattctcag aatgacttgg ttgagtactc 4320
   accagtcaca gaaaagcatc ttacggatgg catgacagta agagaattat gcagtgctgc 4380
   cataaccatg agtgataaca ctgcggccaa cttacttctg acaacgatcg gaggaccgaa 4440
   ggagctaacc gcttttttgc acaacatggg ggatcatgta actcgccttg atcgttggga 4500
   accggagctg aatgaagcca taccaaacga cgagcgtgac accacgatgc ctgtagcaat 4560
   ggcaacaacg ttgcgcaaac tattaactgg cgaactactt actctagctt cccggcaaca 4620
   attaatagac tggatggagg cggataaagt tgcaggacca cttctgcgct cggcccttcc 4680
   ggctggctgg tttattgctg ataaatctgg agccggtgag cgtgggtctc gcggtatcat 4740
   tgcagcactg gggccagatg gtaagccctc ccgtatcgta gttatctaca cgacggggag 4800
   tcaggcaact atggatgaac gaaatagaca gatcgctgag ataggtgcct cactgattaa 4860
   gcattggtaa ctgtcagacc aagtttactc atatatactt tagattgatt taaaacttca 4920
   tttttaattt aaaaggatct aggtgaagat cctttttgat aatctcatga ccaaaatccc 4980
   ttaacgtgag ttttcgttcc actgagcgtc agaccccgta gaaaagatca aaggatcttc 5040
   ttgagatcct ttttttctgc gcgtaatctg ctgcttgcaa acaaaaaaac caccgctacc 5100
   agcggtggtt tgtttgccgg atcaagagct accaactctt tttccgaagg taactggctt 5160
   cagcagagcg cagataccaa atactgtcct tctagtgtag ccgtagttag gccaccactt 5220
   caagaactct gtagcaccgc ctacatacct cgctctgcta atcctgttac cagtggctgc 5280
   tgccagtggc gataagtcgt gtcttaccgg gttggactca agacgatagt taccggataa 5340
   ggcgcagcgg tcgggctgaa cggggggttc gtgcacacag cccagcttgg agcgaacgac 5400
   ctacaccgaa ctgagatacc tacagcgtga gctatgagaa agcgccacgc ttcccgaagg 5460
   gagaaaggcg gacaggtatc cggtaagcgg cagggtcgga acaggagagc gcacgaggga 5520
   gcttccaggg ggaaacgcct ggtatcttta tagtcctgtc gggtttcgcc acctctgact 5580
   tgagcgtcga tttttgtgat gctcgtcagg ggggcggagc ctatggaaaa acgccagcaa 5640
   cgcggccttt ttacggttcc tggccttttg ctggcctttt gctcacatgt tctttcctgc 5700
   gttatcccct gattctgtgg ataaccgtat taccgccttt gagtgagctg ataccgctcg 5760
   ccgcagccga acgaccgagc gcagcgagtc agtgagcgag gaagcggaag agcgcccaat 5820
   acgcaaaccg cctctccccg cgcgttggcc gattcattaa tgcagctggc acgacaggtt 5880
   tcccgactgg aaagcgggca gtgagcgcaa cgcaattaat gtgagttagc tcactcatta 5940
   ggcaccccag gctttacact ttatgcttcc ggctcgtatg ttgtgtggaa ttgtgagcgg 6000
   ataacaattt cacacaggaa acagctatga ccatgattac gccaagcgcg caattaaccc 6060
   tcactaaagg gaacaaaagc t 6081
<210> 18
   <211> 34
   <212> DNA
   <213> artificial sequence
<220>
   <223> Lox 5171
<400> 18
   ataacttcgt ataatgtgta ctatacgaag ttat 34
<210> 19
   <211> 34
   <212> DNA
   <213> artificial sequence
<220>
   <223> Lox 5271
<400> 19
   ataacttcgt ataatgttta ctatacgaag ttat 34
<210> 20
   <211> 34
   <212> DNA
   <213> artificial sequence
<220>
   <223> Lox 5371
<400> 20
   ataacttcgt ataatgtcta ctatacgaag ttat 34
<210> 21
   <211> 34
   <212> DNA
   <213> artificial sequence
<220>
   <223> Lox 5172
<400> 21
   ataacttcgt ataatgtgtc ctatacgaag ttat 34
<210> 22
   <211> 34
   <212> DNA
   <213> artificial sequence
<220>
   <223> Lox 5272
<400> 22
   ataacttcgt ataatgtttc ctatacgaag ttat 34
<210> 23
   <211> 34
   <212> DNA
   <213> artificial sequence
<220>
   <223> Lox 5372
<400> 23
   ataacttcgt ataatgtctc ctatacgaag ttat 34
<210> 24
   <211> 34
   <212> DNA
   <213> artificial sequence
<220>
   <223> m2
<400> 24
   ataacttcgt ataagaaacc atatacgaag ttat 34
<210> 25
   <211> 34
   <212> DNA
   <213> artificial sequence
<220>
   <223> m3
<400> 25
   ataacttcgt atataatacc atatacgaag ttat 34
<210> 26
   <211> 34
   <212> DNA
   <213> artificial sequence
<220>
   <223> m7
<400> 26
   ataacttcgt ataagataga atatacgaag ttat 34
<210> 27
   <211> 34
   <212> DNA
   <213> artificial sequence
<220>
   <223> m11
<400> 27
   ataacttcgt atacgatacc atatacgaag ttat 34
<210> 28
   <211> 23
   <212> DNA
   <213> artificial sequence
<220>
   <223> pgk-F
<400> 28
   cacgcttcaa aagcgcacgt ctg 23
<210> 29
   <211> 25
   <212> DNA
   <213> artificial sequence
<220>
   <223> neo-R
<400> 29
   gttgtgccca gtcatagccg aatag 25
<210> 30
   <211> 28
   <212> DNA
   <213> artificial sequence
<220>
   <223> Hyg-1
<400> 30
   gaagaatctc gtgctttcag cttcgatg 28
<210> 31
   <211> 25
   <212> DNA
   <213> artificial sequence
<220>
   <223> Hyg-2
<400> 31
   aatgaccgct gttatgcggc cattg 25
<210> 32
   <211> 20
   <212> DNA
   <213> artificial sequence
<220>
   <223> Rosa-5'
<400> 32
   cgtgttcgtg caagttgagt 20
<210> 33
   <211> 19
   <212> DNA
   <213> artificial sequence
<220>
   <223> Rosa-3'
<400> 33
   actcccgccc atcttctag 19
<210> 34
   <211> 19
   <212> DNA
   <213> artificial sequence
<220>
   <223> Probe-F
<400> 34
   aaggatactg gggcatacg 19
<210> 35
   <211> 23
   <212> DNA
   <213> artificial sequence
<220>
   <223> Probe-R
<400> 35
   cttctcagct acctttacac acc 23